# EUROPÄISCHE PATENTSCHRIFT

(11) **EP 3 161 118 B1**
(45) Veröffentlichungstag und Bekanntmachung des Hinweises auf die Patenterteilung: **07.08.2019**
(21) Anmeldenummer: 15730147.4
(22) Anmeldetag: 18.06.2015
(51) Int. Cl.: C11D 3/386

(54) **REINIGUNGSMITTEL ENTHALTEND AMYLASEN**
CLEANING AGENT CONTAINING AMYLASES
PRODUIT DE NETTOYAGE CONTENANT DES AMYLASES

(30) Priorität: 30.06.2014 DE 102014212640
(43) Veröffentlichungstag der Anmeldung: 03.05.2017
(73) Patentinhaber: Henkel AG & Co. KGaA, 40589 Düsseldorf (DE)
(72) Erfinder: VOCKENROTH, Inga Kerstin, 40597 Düsseldorf (DE); WEBER, Thomas, 41541 Dormagen (DE); MENKE, Silke, 42781 Haan (DE); EITING, Thomas, 40589 Düsseldorf (DE); MUSSMANN, Nina, 47877 Willich (DE); BASTIGKEIT, Thorsten, 42279 Wuppertal (DE); WRUBBEL, Noelle, 40591 Düsseldorf (DE)
(86) Internationale Anmeldenummer: PCT/EP2015/063667
(87) Internationale Veröffentlichungsnummer: WO 2016/000972

(56) Entgegenhaltungen:
- EP-A1- 2 100 949
- WO-A1-2010/090915
- US-A1- 2014 179 585

### SEQUENCE LISTING

<110> Henkel AG & Co. KGaA
<120> Reinigungsmittel enthaltend Amylasen
<130> PT032540PCT
<150> 102014212640,1
   <151> 2014-06-30
<160> 8
<170> PatentIn version 3.5
<210> 1
   <211> 485
   <212> PRT
   <213> Bacillus sp. No.707
<400> 1
   His His Asn Gly Thr Asn Gly Thr Met Met Gln Tyr Phe Glu Trp Tyr
   1               5                   10                  15
   Leu Pro Asn Asp Gly Asn His Trp Asn Arg Leu Asn Ser Asp Ala Ser
               20                  25                  30
   Asn Leu Lys Ser Lys Gly Ile Thr Ala Val Trp Ile Pro Pro Ala Trp
           35                  40                  45
   Lys Gly Ala Ser Gln Asn Asp Val Gly Tyr Gly Ala Tyr Asp Leu Tyr
       50                  55                  60
   Asp Leu Gly Glu Phe Asn Gln Lys Gly Thr Val Arg Thr Lys Tyr Gly
   65                  70                  75                  80
   Thr Arg Ser Gln Leu Gln Ala Ala Val Thr Ser Leu Lys Asn Asn Gly
                   85                  90                  95
   Ile Gln Val Tyr Gly Asp Val Val Met Asn His Lys Gly Gly Ala Asp
               100                 105                 110
   Ala Thr Glu Met Val Arg Ala Val Glu Val Asn Pro Asn Asn Arg Asn
           115                 120                 125
   Gln Glu Val Thr Gly Glu Tyr Thr Ile Glu Ala Trp Thr Arg Phe Asp
       130                 135                 140
   Phe Pro Gly Arg Gly Asn Thr His Ser Ser Phe Lys Trp Arg Trp Tyr
   145                 150                 155                 160
   His Phe Asp Gly Val Asp Trp Asp Gln Ser Arg Arg Leu Asn Asn Arg
   165 170 175
   Ile Tyr Lys Phe Arg Gly His Gly Lys Ala Trp Asp Trp Glu Val Asp
   180 185 190
   Thr Glu Asn Gly Asn Tyr Asp Tyr Leu Met Tyr Ala Asp Ile Asp Met
   195 200 205
   Asp His Pro Glu Val Val Asn Glu Leu Arg Asn Trp Gly Val Trp Tyr
   210 215 220
   Thr Asn Thr Leu Gly Leu Asp Gly Phe Arg Ile Asp Ala Val Lys His
   225 230 235 240
   Ile Lys Tyr Ser Phe Thr Arg Asp Trp Ile Asn His Val Arg Ser Ala
   245 250 255
   Thr Gly Lys Asn Met Phe Ala Val Ala Glu Phe Trp Lys Asn Asp Leu
   260 265 270
   Gly Ala Ile Glu Asn Tyr Leu Gln Lys Thr Asn Trp Asn His Ser Val
   275 280 285
   Phe Asp Val Pro Leu His Tyr Asn Leu Tyr Asn Ala Ser Lys Ser Gly
   290 295 300
   Gly Asn Tyr Asp Met Arg Asn Ile Phe Asn Gly Thr Val Val Gln Arg
   305 310 315 320
   His Pro Ser His Ala Val Thr Phe Val Asp Asn His Asp Ser Gln Pro
   325 330 335
   Glu Glu Ala Leu Glu Ser Phe Val Glu Glu Trp Phe Lys Pro Leu Ala
   340 345 350
   Tyr Ala Leu Thr Leu Thr Arg Glu Gln Gly Tyr Pro Ser Val Phe Tyr
   355 360 365
   Gly Asp Tyr Tyr Gly Ile Pro Thr His Gly Val Pro Ala Met Arg Ser
   370 375 380
   Lys Ile Asp Pro Ile Leu Glu Ala Arg Gln Lys Tyr Ala Tyr Gly Lys
   385 390 395 400
   Gln Asn Asp Tyr Leu Asp His His Asn Ile Ile Gly Trp Thr Arg Glu
   405 410 415
   Gly Asn Thr Ala His Pro Asn Ser Gly Leu Ala Thr Ile Met Ser Asp
   420 425 430
   Gly Ala Gly Gly Ser Lys Trp Met Phe Val Gly Arg Asn Lys Ala Gly
   435 440 445
   Gln Val Trp Ser Asp Ile Thr Gly Asn Arg Thr Gly Thr Val Thr Ile
   450 455 460
   Asn Ala Asp Gly Trp Gly Asn Phe Ser Val Asn Gly Gly Ser Val Ser
   465 470 475 480
   Ile Trp Val Asn Lys
   485
<210> 2
   <211> 485
   <212> PRT
   <213> Bacillus sp.
<400> 2
   His His Asn Gly Thr Asn Gly Thr Met Met Gln Tyr Phe Glu Trp Tyr
   1 5 10 15
   Leu Pro Asn Asp Gly Asn His Trp Asn Arg Leu Arg Ser Asp Ala Ser
   20 25 30
   Asn Leu Lys Asp Lys Gly Ile Ser Ala Val Trp Ile Pro Pro Ala Trp
   35 40 45
   Lys Gly Ala Ser Gln Asn Asp Val Gly Tyr Gly Ala Tyr Asp Leu Tyr
   50 55 60
   Asp Leu Gly Glu Phe Asn Gln Lys Gly Thr Ile Arg Thr Lys Tyr Gly
   65 70 75 80
   Thr Arg Asn Gln Leu Gln Ala Ala Val Asn Ala Leu Lys Ser Asn Gly
   85 90 95
   Ile Gln Val Tyr Gly Asp Val Val Met Asn His Lys Gly Gly Ala Asp
   100 105 110
   Ala Thr Glu Met Val Arg Ala Val Glu Val Asn Pro Asn Asn Arg Asn
   115 120 125
   Gln Glu Val Ser Gly Glu Tyr Thr Ile Glu Ala Trp Thr Lys Phe Asp
   130 135 140
   Phe Pro Gly Arg Gly Asn Thr His Ser Asn Phe Lys Trp Arg Trp Tyr
   145 150 155 160
   His Phe Asp Gly Val Asp Trp Asp Gln Ser Arg Lys Leu Asn Asn Arg
   165 170 175
   Ile Tyr Lys Phe Arg Gly Asp Gly Lys Gly Trp Asp Trp Glu Val Asp
   180 185 190
   Thr Glu Asn Gly Asn Tyr Asp Tyr Leu Met Tyr Ala Asp Ile Asp Met
   195 200 205
   Asp His Pro Glu Val Val Asn Glu Leu Arg Asn Trp Gly Val Trp Tyr
   210 215 220
   Thr Asn Thr Leu Gly Leu Asp Gly Phe Arg Ile Asp Ala Val Lys His
   225 230 235 240
   Ile Lys Tyr Ser Phe Thr Arg Asp Trp Ile Asn His Val Arg Ser Ala
   245 250 255
   Thr Gly Lys Asn Met Phe Ala Val Ala Glu Phe Trp Lys Asn Asp Leu
   260 265 270
   Gly Ala Ile Glu Asn Tyr Leu Asn Lys Thr Asn Trp Asn His Ser Val
   275 280 285
   Phe Asp Val Pro Leu His Tyr Asn Leu Tyr Asn Ala Ser Lys Ser Gly
   290 295 300
   Gly Asn Tyr Asp Met Arg Gln Ile Phe Asn Gly Thr Val Val Gln Arg
   305 310 315 320
   His Pro Met His Ala Val Thr Phe Val Asp Asn His Asp Ser Gln Pro
   325 330 335
   Glu Glu Ala Leu Glu Ser Phe Val Glu Glu Trp Phe Lys Pro Leu Ala
   340 345 350
   Tyr Ala Leu Thr Leu Thr Arg Glu Gln Gly Tyr Pro Ser Val Phe Tyr
   355 360 365
   Gly Asp Tyr Tyr Gly Ile Pro Thr His Gly Val Pro Ala Met Lys Ser
   370 375 380
   Lys Ile Asp Pro Ile Leu Glu Ala Arg Gln Lys Tyr Ala Tyr Gly Arg
   385 390 395 400
   Gln Asn Asp Tyr Leu Asp His His Asn Ile Ile Gly Trp Thr Arg Glu
   405 410 415
   Gly Asn Thr Ala His Pro Asn Ser Gly Leu Ala Thr Ile Met Ser Asp
   420 425 430
   Gly Ala Gly Gly Asn Lys Trp Met Phe Val Gly Arg Asn Lys Ala Gly
   435 440 445
   Gln Val Trp Thr Asp Ile Thr Gly Asn Arg Ala Gly Thr Val Thr Ile
   450 455 460
   Asn Ala Asp Gly Trp Gly Asn Phe Ser Val Asn Gly Gly Ser Val Ser
   465 470 475 480
   Ile Trp Val Asn Lys
   485
<210> 3
   <211> 269
   <212> PRT
   <213> Bacillus lentus
<400> 3
   Ala Gln Ser Val Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Ser Gly Ser Gly Ser Val Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Gly Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 4
   <211> 269
   <212> PRT
   <213> Bacillus lentus
<400> 4
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 5
   <211> 269
   <212> PRT
   <213> Artificial
<220>
   <223> Optimized B. lentus subtilisin 309 variant
<400> 5
   Ala Gln Ser Val Pro Trp Gly Ile Arg Arg Val Gln Ala Pro Thr Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Ala Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ser Gly Ser Val Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Gly Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asp Gly Thr Ser Met Ala Thr Pro His Val Ala Gly Ala
       210                 215                 220
   Ala Ala Leu Val Lys Gln Lys Asn Pro Ser Trp Ser Asn Val Arg Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 6
   <211> 270
   <212> PRT
   <213> Artificial
<220>
   <223> Optimized B. lentus subtilisin 309 variant
<400> 6
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ala Asp Gly Ser Val Ser Ser Ile Ala Gln Gly Leu Glu Trp
               100                 105                 110
   Ala Gly Asn Asn Gly Met His Val Ala Asn Leu Ser Leu Gly Ser Pro
           115                 120                 125
   Ser Pro Ser Ala Thr Leu Glu Gln Ala Val Asn Ser Ala Thr Ser Arg
       130                 135                 140
   Gly Val Leu Val Val Ala Ala Ser Gly Asn Ser Gly Ala Gly Ser Ile
   145                 150                 155                 160
   Ser Tyr Pro Ala Arg Tyr Ala Asn Ala Met Ala Val Gly Ala Thr Asp
                   165                 170                 175
   Gln Asn Asn Asn Arg Ala Ser Phe Ser Gln Tyr Gly Ala Gly Leu Asp
               180                 185                 190
   Ile Val Ala Pro Gly Val Asn Val Gln Ser Thr Tyr Pro Gly Ser Thr
           195                 200                 205
   Tyr Ala Ser Leu Asn Gly Thr Ser Met Ala Thr Pro His Val Ala Gly
       210                 215                 220
   Ala Ala Ala Leu Val Lys Gln Lys Asn Pro Ser Trp Ser Asn Val Gln
   225                 230                 235                 240
   Ile Arg Asn His Leu Lys Asn Thr Ala Thr Ser Leu Gly Ser Thr Asn
                   245                 250                 255
   Leu Tyr Gly Ser Gly Leu Val Asn Ala Glu Ala Ala Thr Arg
               260                 265                 270
<210> 7
   <211> 269
   <212> PRT
   <213> Artificial
<220>
   <223> Optimized B. lentus alkaline protease variant
<400> 7
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Glu Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 8
   <211> 269
   <212> PRT
   <213> Artificial
<220>
   <223> Optimized B. lentus alkaline protease variant
<400> 8
   Ala Gln Thr Ile Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Glu Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Ile Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265

## Beschreibung

Die vorliegende Anmeldung richtet sich auf ein Reinigungsmittel, bevorzugt ein Geschirrspülmittel, vorzugsweise ein maschinelles Geschirrspülmittel, das mindestens zwei Amylasen und mindestens eine Protease enthält, sowie die Verwendung eines solchen Reinigungsmittels.

Das wichtigste Kriterium beim Reinigen von Textilien, harten Oberflächen, wie insbesondere beim Geschirrspülen, insbesondere beim maschinellen Geschirrspülen, ist die Reinigungsleistung an verschiedensten Anschmutzungen, welche insbesondere in Form von Lebensmittelresten eingebracht werden. Aus EP 2 100 949 A1, WO 2010 090 915 A1 und US 2014 0 179 585 A1 sind Geschirrspülmittel bekannt, in denen eine Kombination aus Amylase und Protease eingesetzt wird.

Auch wenn die Reinigungsleistung der heutzutage eingesetzten Geschirrspülmittel generell hoch ist, stellt sich, auch aufgrund des generellen Trends beim maschinellen Geschirrspülen vermehrt Niedrigtemperatur-Programme zu verwenden, allerdings das Problem, dass viele der üblichen maschinellen Geschirrspülmittel bei hartnäckigen Anschmutzungen eine unzureichende Reinigungsleistung aufweisen. Eine solche unzureichende Reinigungsleistung und die damit einhergehende unzureichende Reinigung des Geschirrs führen beim Verbraucher zu Unzufriedenheit und dazu, dass solche Anschmutzungen vom Verbraucher vorbehandelt werden, was wiederum den Wasser- und Energieverbrauch erhöht. Daher besteht ein allgemeiner Bedarf nach maschinellen Geschirrspülmitteln, die auch an hartnäckigen, insbesondere angebrannten Anschmutzungen noch eine gute Reinigungsleistung aufweisen, ohne dabei die bestehende gute Reinigungsleistung an anderen Anschmutzungen zu verringern.

Die Aufgabe der vorliegenden Erfindung bestand daher darin, ein Reinigungsmittel, bevorzugt ein Geschirrspülmittel, vorzugsweise ein maschinelles Geschirrspülmittel, zur Verfügung zu stellen, das bei solchen Anschmutzungen eine gesteigerte Reinigungsleistung aufweist, ohne dass die Reinigungsleistung an anderen Anschmutzungen verringert wird.

Überraschenderweise wurde nun festgestellt, dass die Verwendung einer Kombination verschiedener Amylasen die Reinigungsleistung von entsprechenden Reinigungsmitteln, bevorzugt eines Geschirrspülmittels, vorzugsweise eines maschinellen Geschirrspülmittels an enzym-sensitiven Anschmutzungen, insbesondere angebrannten Lebensmittelanschmutzungen, verbessert.

In einem ersten Aspekt richtet sich die vorliegende Erfindung daher auf ein Reinigungsmittel (für harte Oberflächen), insbesondere ein Geschirrspülmittel, vorzugsweise ein maschinelles Geschirrspülmittel, das eine erste und eine zweite Amylase umfasst, wobei
a) die erste Amylase eine α-Amylase aus Bacillus sp. No. 707 oder ein funktionales Fragment oder eine Variante davon ist;
b) die zweite Amylase eine AA560 α-Amylase aus Bacillus sp. oder ein funktionales Fragment oder eine Variante davon ist, und es ferner mindestens eine Protease enthält.

Eine solche Kombination bewirkt bei Anwendung des Mittels eine signifikant gesteigerte Reinigungsleistung an hartnäckigen Anschmutzungen, insbesondere an stärkehaltigen Anschmutzungen.

Ein weiterer Gegenstand der vorliegenden Erfindung ist die Verwendung eines hierin beschriebenen Reinigungsmittels, bevorzugt eines Geschirrspülmittels, vorzugsweise eines maschinellen Geschirrspülmittels in einem Reinigungsverfahren, bevorzugt einem Geschirrspülverfahren, insbesondere in einem maschinellen Geschirrspülverfahren, vorzugsweise die Verwendung zur Verbesserung der Reinigungsleistung, insbesondere der Reinigungsleistung an enzym-sensitiven Anschmutzungen, an harten Oberflächen, insbesondere Geschirr bei dessen Reinigung, bevorzugt in einer automatischen Geschirrspülmaschine, insbesondere hartnäckigen Anschmutzungen, u.a. auch bei Temperaturen, die niedriger sind als die üblicherweise verwendeten Temperaturen.

Ein weiterer Gegenstand der vorliegenden Erfindung ist ein Reinigungsverfahren, bevorzugt Geschirrspülverfahren, vorzugsweise ein maschinelles Geschirrspülverfahren, bei dem ein hierin beschriebenes Reinigungsmittel, bevorzugt ein Geschirrspülmittel, vorzugsweise ein maschinelles Geschirrspülmittel, insbesondere zu dem Zweck, die Reinigungsleistung an enzym-sensitiven Anschmutzungen zu verbessern, zum Einsatz kommt. In verschiedenen Ausführungsformen der Erfindung werden bei dem Geschirrspülverfahren Temperaturen eingesetzt, die niedriger sind als die üblicherweise verwendeten Temperaturen.

Noch ein weiterer Gegenstand der Erfindung ist die Verwendung der hierin beschriebenen Enzymkombinationen zur Verbesserung der Reinigungsleistung eines Reinigungsmittels, insbesondere eines Geschirrspülmittels.

"Niedrige Temperaturen" oder "Temperaturen, die niedriger sind als die üblicherweise verwendeten Temperaturen", wie hierin im Zusammenhang mit Geschirrspülverfahren verwendet, bezieht sich vorzugsweise auf Temperaturen unter 60 °C, insbesondere unter 55 °C, noch bevorzugter 50 °C oder niedriger, besonders bevorzugt 45 °C oder niedriger und am bevorzugtesten 40°C oder niedriger. Diese Temperaturangaben beziehen sich auf die in den Reinigungsschritten eingesetzten Zieltemperaturen (Höchsttemperaturen).

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist. Alle Prozentangaben sind, sofern nicht anders angegeben, Gewichts-%. Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

Die eingesetzten Amylasen sind alkalische α-Amylasen. Sie wirken als Hydrolasen und spalten die α(1-4)-Glykosidbindung von Polysacchariden, insbesondere Stärken wie Amylose, und bewirken dadurch den Abbau stärkehaltiger Anschmutzungen auf dem Reinigungsgut. Als Spaltprodukte entstehen dabei Dextrine und daraus Maltose, Glucose und verzweigte Oligosaccharide. Ihr pH-Optimum liegt meist im deutlich alkalischen Bereich.

Die Wildttyp-Sequenzen der reifen (maturen) α-Amylase aus Bacillus sp. No. 707 bzw. der reifen (maturen) AA560 α-Amylase aus Bacillus sp. sind in SEQ ID NO:1 bzw. SEQ ID NO:2 angegeben.

"Verschieden", wie hierin mit Bezug auf die Enzyme verwendet, bezieht sich auf Enzyme, die sich in ihrer Aminosäuresequenz unterscheiden. In verschiedenen Ausführungsformen stammen voneinander verschiedene Enzyme aus unterschiedlichen Arten von Organismen oder unterscheiden sich voneinander durch, beispielsweise künstlich erzeugte, Mutationen.

"Variante", wie hierin in Zusammenhang mit Enzymen verwendet, bezieht sich auf natürliche oder artifiziell erzeugte Variationen eines nativen Enzyms, die eine gegenüber der Referenzform abgewandelte Aminosäuresequenz aufweisen. Eine solche Variante kann einzelne oder mehrere Punktmutationen, d.h. Substitutionen einer natürlicherweise an der entsprechenden Position vorkommenden Aminosäure durch eine andere, Insertionen (Einfügen von einer oder mehreren Aminosäuren) und/oder Deletionen (Entfernen von einer oder mehreren Aminosäuren) aufweisen, insbesondere eine oder mehrere Punktmutationen. Derartige Varianten haben vorzugsweise mindestens 50, vorzugsweise 60 oder mehr, noch bevorzugter 70, 80, 90, 100 % oder mehr der Enzymaktivität der Referenzform. In verschiedenen Ausführungsformen hat eine derartige Variante eine Aminosäuresequenz, die zu der als Referenz dienenden Sequenz über deren Gesamtlänge zu mindestens 70, vorzugsweise 75, 80, 85, 90, 95, 96, 97, 98, oder 99 % identisch ist. Die Varianten haben vorzugsweise dieselbe Länge wie die Referenzsequenz. Varianten können sich gegenüber der Referenzform durch verbesserte Eigenschaften auszeichnen, wie beispielsweise höhere Enzymaktivität, höhere Stabilität, geänderte Substratspezifität, etc.. Eingesetzt werden nur solche Varianten, die enzymatische Aktivität aufweisen. "Enzymatische Aktivität", wie in diesem Zusammenhang verwendet, bedeutet insbesondere, dass die entsprechenden Enzyme mindestens 50 %, vorzugsweise mindestens 90 % der katalytischen Aktivität ihres Referenzenzymes aufweisen.

"Fragment", wie hierin in Zusammenhang mit Enzymen verwendet, bezieht sich auf gegenüber dem Referenz-Enzym N-terminal und/oder C-terminal um jeweils eine oder mehrere Aminosäuren verkürzte Polypeptide. Eingesetzt werden nur solche Fragmente, die enzymatische Aktivität aufweisen. "Enzymatische Aktivität", wie in diesem Zusammenhang verwendet, bedeutet insbesondere, dass die entsprechenden Enzyme mindestens 50 %, vorzugsweise mindestens 90 % der katalytischen Aktivität ihres Referenzenzymes aufweisen.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Als erste Amylase im Sinne der vorliegenden Erfindung wird in verschiedenen bevorzugten Ausführungsformen eine Amylase eingesetzt, die eine Aminosäuresequenz umfasst, die zu der in SEQ ID NO. 1 angegebenen Aminosäuresequenz über deren Gesamtlänge zu mindestens 80% identisch ist und optional mindestens eine Aminosäuresubstitution an einer der Positionen 172, 202, 208, 255 und 261 in der Zählung gemäß SEQ ID NO. 1 aufweist, insbesondere ausgewählt aus der Gruppe bestehend aus M202L, M202V, M202S, M202T, M202I, M202Q, M202W, S255N und R172Q.

In bevorzugten Ausführungsformen der Erfindung wird somit als erste Amylase eine Variante von der α-Amylase aus Bacillus sp. No. 707 mit der in SEQ ID NO:1 angegebenen Aminosäuresequenz eingesetzt, die zu der in SEQ ID NO. 1 angegebenen Aminosäuresequenz über deren Gesamtlänge zu mindestens 80% identisch ist und mindestens eine Aminosäuresubstitution an einer der Positionen 172, 202, 208, 255 und 261 in der Zählung gemäß SEQ ID NO. 1 aufweist. Bevorzugt werden Amylasen eingesetzt, die an zwei, bevorzugt drei, der vorstehend genannten Positionen eine Aminosäuresubstitution aufweisen, insbesondere eine Substitution an Position 202 ausgewählt aus M202L, M202V, M202S, M202T, M202I, M202Q, M202W, eine Substitution an Position 255, insbesondere S255N, und eine Substitution an Position 172, insbesondere R172Q. Ganz besonders bevorzugt sind die M202L und M202T Mutanten.

Weitere einsetzbare Varianten sind solche, die eine Aminosäuresequenz besitzen, die zu der in SEQ ID NO. 1 angegebenen Aminosäuresequenz mindestens zu 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% oder 99% identisch ist, wobei die Positionen 172, 202 und 255 vorzugsweise wie oben beschrieben substituiert sind. Derartige Varianten können beispielsweise eine Verkürzung des C-Terminus einschließen, beispielsweise um 1-20 Aminosäure, oder eine Deletion von einer oder mehreren Aminosäuren, insbesondere an den Positionen 181, 182, 183 und 184 in der Zählung gemäß SEQ ID NO:1, wobei allerdings die enzymatische Aktivität beibehalten wird, d.h. die Aktivität der Variante mindestens 60 % der Aktivität des Enzyms mit der Aminosäuresequenz von SEQ ID NO:1 beträgt. Geeignete Amylasen werden auch in der WO 2008/112459 A2 beschrieben, deren gesamte Offenbarung hierin durch Bezugnahme eingeschlossen ist.

Die zweite Amylase ist von der ersten Amylase verschieden, d. h. eine Amylase, die sowohl unter die Definition für die erste Amylase als auch für die der zweiten Amylase fällt, kann nicht als erste und als zweite Amylase gleichzeitig gezählt werden.

Als zweite Amylase im Sinne der vorliegenden Erfindung wird in verschiedenen bevorzugten Ausführungsformen eine Amylase eingesetzt, die eine Aminosäuresequenz umfasst, die zu der in SEQ ID NO: 2 angegebenen Aminosäuresequenz über deren Gesamtlänge zu mindestens 80% identisch ist und optional mindestens eine Aminosäuresubstitution an einer der Positionen 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 und 484 und/oder eine der Deletionen D183* and G184* in der Zählung gemäß SEQ ID NO:2 aufweist.

In verschiedenen bevorzugten Ausführungsformen weist die zweite Amylase in der Zählung gemäß SEQ ID NO: 2 Aminosäuresubstitutionen an drei oder mehr der Positionen 9, 26, 149, 182, 186, 202, 257, 295, 299, 323, 339 and 345 und optional eine oder mehrere, vorzugsweise alle, der Substitutionen und/oder Deletionen an den Positionen: 118, 183, 184, 195, 320 und 458, besonders bevorzugt R118K, D183*, G184*, N195F, R320K und/oder R458K, auf.

In besonders bevorzugten Ausführungsformen weist zweite Amylase in der Zählung gemäß SEQ ID NO:2 folgende Aminosäuresubstitutionen und/oder Deletionen auf:
(i) M9L + M323T;
(ii) M9L + M202L/T/V/I + M323T;
(iii) M9L + N195F + M202L/T/V/I + M323T;
(iv) M9L + R118K + D183* + G184* + R320K + M323T + R458K;
(v) M9L + R118K + D183* + G184* + M202L/T/V/I + R320K + M323T + R458K;
(vi) M9L + G149A + G182T + G186A + M202L + T257I + Y295F + N299Y + M323T + A339S + E345R;
(vii) M9L + G149A + G182T + G186A + M202I + T257I + Y295F + N299Y + M323T + A339S + E345R;
(viii) M9L + R118K + G149A + G182T + D183* + G184* + G186A + M202L + T257I + Y295F + N299Y + R320K + M323T + A339S + E345R + R458K;
(ix) M9L + R118K + G149A + G182T + D183* + G184* + G186A + N195F + M202L + T257I + Y295F + N299Y + R320K + M323T + A339S + E345R + R458K;
(x) M9L + R118K + G149A + G182T + D183* + G184* + G186A + M202I + T257I + Y295F + N299Y + R320K + M323T + A339S + E345R + R458K;
(xi) M9L + R118K + D183* + D184* + N195F + M202L + R320K + M323T + R458K;
(xii) M9L + R118K + D183* + D184* + N195F + M202T + R320K + M323T + R458K;
(xiii) M9L + R118K + D183* + D184* + N195F + M202I + R320K + M323T + R458K;
(xiv) M9L + R118K + D183* + D184* + N195F + M202V + R320K + M323T + R458K;
(xv) M9L + R118K + N150H + D183* + D184* + N195F + M202L + V214T + R320K + M323T + R458K; oder
(xvi) M9L + R118K + D183* + D184* + N195F + M202L + V214T + R320K + M323T + E345N + R458K.

Eine besonders bevorzugte zweite Amylase ist die Variante, die unter dem Handelnamen Stainzyme Plus™ kommerziell erhältlich ist (Novozymes A/S, Bagsvaerd, Dänemark).

Bevorzugt werden im Rahmen der vorliegenden Erfindung Kombinationen einer ersten Amylase, die zu der in SEQ ID NO:1 angegebenen Aminosäuresequenz über deren Gesamtlänge zu mindestens 80% identisch ist und mindestens eine Aminosäuresubstitution an einer der Positionen 172, 202, 208, 255 und 261 in der Zählung gemäß SEQ ID NO:1 aufweist, insbesondere eine Substitution an Position 202 ausgewählt aus M202L, M202V, M202S, M202T, M202I, M202Q, M202W, eine Substitution an Position 255, insbesondere S255N, und eine Substitution an Position 172, insbesondere R172Q, und einer zweiten Amylase, die zu der in SEQ ID NO:2 angegebenen Aminosäuresequenz über deren Gesamtlänge zu mindestens 80% identisch ist und folgende Aminosäuresubstitutionen und/oder Deletionen aufweist
(i) M9L + M323T;
(ii) M9L + M202L/T/V/I + M323T;
(iii) M9L + N195F + M202L/T/V/I + M323T;
(iv) M9L + R118K + D183* + G184* + R320K + M323T + R458K;
(v) M9L + R118K + D183* + G184* + M202L/T/V/I + R320K + M323T + R458K;
(vi) M9L + G149A + G182T + G186A + M202L + T257I + Y295F + N299Y + M323T + A339S + E345R;
(vii) M9L + G149A + G182T + G186A + M202I + T257I + Y295F + N299Y + M323T + A339S + E345R;
(viii) M9L + R118K + G149A + G182T + D183* + G184* + G186A + M202L + T257I + Y295F + N299Y + R320K + M323T + A339S + E345R + R458K;
(ix) M9L + R118K + G149A + G182T + D183* + G184* + G186A + N195F + M202L + T257I + Y295F + N299Y + R320K + M323T + A339S + E345R + R458K;
(x) M9L + R118K + G149A + G182T + D183* + G184* + G186A + M202I + T257I + Y295F + N299Y + R320K + M323T + A339S + E345R + R458K;
(xi) M9L + R118K + D183* + D184* + N195F + M202L + R320K + M323T + R458K;
(xii) M9L + R118K + D183* + D184* + N195F + M202T + R320K + M323T + R458K;
(xiii) M9L + R118K + D183* + D184* + N195F + M202I + R320K + M323T + R458K;
(xiv) M9L + R118K + D183* + D184* + N195F + M202V + R320K + M323T + R458K;
(xv) M9L + R118K + N150H + D183* + D184* + N195F + M202L + V214T + R320K + M323T + R458K; oder
(xvi) M9L + R118K + D183* + D184* + N195F + M202L + V214T + R320K + M323T + E345N + R458K.

In verschiedenen Ausführungsformen werden diese Kombinationen von Amylasen im Massenverhältnis von 50:1 bis 1:50, vorzugsweise 30:1 bis 1:10 (jeweils bezogen auf die Menge Aktivprotein Amylase 1 zu Amylase 2) eingesetzt.

Es ist insbesondere bevorzugt, eine Amylase gemäß 1) im Verhältnis zu einer Amylase gemäß 2) in einem Verhältnis 20:1 bis 2:1, bevorzugt 15:1 bis 3:1, besonders bevorzugt 12:1 bis 5:1, beispielsweise 10:1 einzusetzen. Insbesondere ist es in diesem Zusammenhang ganz bevorzugt, als erste Amylase eine der im vorstehend genannten Varianten i) bis xvi) der SeqID 1 und als zweite Amylase eine der im vorstehend genannten Varianten i) bis xvi) der SeqID 2 in einem Verhältnis von 15:1 bis 3:1, besonders bevorzugt 12:1 bis 5:1, beispielsweise 10:1 einzusetzen.

Die hierin beschriebenen Reinigungsmittel enthalten in bevorzugten Ausführungsformen ferner mindestens eine Protease, insbesondere mindestens eine erste und mindestens eine zweite Protease.

Die Proteasen sind insbesondere alkalische Serin-Proteasen. Sie wirken als unspezifische Endopeptidasen, das heißt, sie hydrolysieren beliebige Säureamidbindungen, die im Inneren von Peptiden oder Proteinen liegen und bewirken dadurch den Abbau proteinhaltiger Anschmutzungen auf dem Reinigungsgut. Ihr pH-Optimum liegt meist im deutlich alkalischen Bereich.

Die mindestens eine Protease wird vorzugsweise ausgewählt aus der Gruppe bestehend aus einem Subtilisin 309 aus Bacillus lentus oder einem funktionalen Fragment oder einer Variante davon und einer alkalischen Protease aus Bacillus lentus DSM 5483 oder einem funktionalen Fragment oder einer Variante davon. Es können ebenfalls Kombinationen von mehreren der vorgenannten Enzyme eingesetzt werden.

Die Sequenzen der reifen (maturen) Protease Subtilisin 309 aus Bacillus lentus bzw. der reifen (maturen) alkalische Protease aus Bacillus lentus DSM 5483 sind in SEQ ID NO:3 bzw. SEQ ID NO:4 angegeben.

In verschiedenen Ausführungsformen der Erfindung umfasst die mindestens eine Protease ein Subtilisin 309 aus Bacillus lentus oder ein funktionales Fragment oder eine Variante davon, umfassend eine Aminosäuresequenz, die zu der in SEQ ID NO:3 angegebenen Aminosäuresequenz über deren Gesamtlänge zu mindestens 80%, bevorzugt zu mindestens 90 %, insbesondere zu 100 % identisch ist und mindestens eine Aminosäuresubstitution an einer der Positionen 9, 15, 66, 212 und 239 in der Zählung gemäß SEQ ID NO:3 aufweist. Bevorzugt sind
solche, die an zwei, bevorzugt drei, insbesondere vier, ganz besonders bevorzugt fünf der vorstehend genannten Positionen eine Aminosäuresubstitution aufweisen.

Weitere einsetzbare Varianten sind solche, die eine Aminosäuresequenz besitzen, die zu der in SEQ ID NO:3 angegebenen Aminosäuresequenz mindestens zu 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% oder 99% identisch ist, wobei eine oder mehrere der Positionen 9, 15, 66, 212 und 239 substituiert sind, d.h. die Aminosäure an diesen Positionen nicht der entsprechenden Aminosäure in SEQ ID NO:3 entspricht.

Besonders bevorzugt verwendet wird eine Variante mit mindestens einer, bevorzugt zwei, insbesondere drei, besonders bevorzugt vier oder ganz besonders bevorzugt 5 der Aminosäuresubstitutionen ausgewählt aus S9R, A15T, V66A, N212D und Q239R bezogen auf die Zählung nach SEQ ID NO:3. Bevorzugt sind die folgenden Kombinationen: S9R+V66A+N212D+Q239R, S9R+A15T+N212D+Q239R, S9R+A15T+V66A+Q239R, S9R+A15T+V66A+N212D, A15T+V66A+N212D+Q239R; S9R+A15T+V66A, S9R+A15T+N212D, S9R+A15T+Q239R, S9R+N212D+Q239R, S9R+V66A+N212D, S9R +V66A+ Q239R, A15T+V66A+N212D, A15T+V66A+Q239R, A15T+N212D+Q239R, V66A+N212D+Q239R; S9R+A15T, S9R+V66A, S9R+N212D, S9R+ Q239R, A15T+V66A, A15T+N212D, A15T+Q239R, V66A+N212D, V66A+Q239R, N212D+Q239R. Eine Variante, die alle vorstehend genannten Änderungen umfasst, hat die in SEQ ID NO:5 angegebene Aminosäuresequenz (S9R+A15T+V66A+N212D+Q239R).

Ebenfalls besonders bevorzugt werden Varianten der Protease mit der in SEQ ID NO:3 angegebenen Aminosäuresequenz, die eine Aminosäuresubstitution an der Position 99 und eine Insertion von einer Aminosäure zwischen den Aminosäuren an Positionen 99 und 100 in der Zählung gemäß SEQ ID NO:3 aufweisen, vorzugsweise ausgewählt aus S99A und/oder S99_G100InsD. Weitere einsetzbare Varianten sind solche, die eine Aminosäuresequenz besitzen, die zu der in SEQ ID NO:3 angegebenen Aminosäuresequenz mindestens zu 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% oder 99% identisch ist, wobei diese Varianten eine oder beide der oben genannten Mutationen an den Positionen 99 und 100 aufweisen. Bevorzugt sind solche, die beide Mutationen aufweisen. Eine derartige Variante, hat die in SEQ ID NO:6 angegebene Aminosäuresequenz.

In verschiedenen noch weiteren Ausführungsformen der Erfindung umfasst die mindestens eine Protease eine alkalische Protease aus Bacillus lentus DSM 5483 oder ein funktionales Fragment oder eine Variante davon, die eine Aminosäuresequenz aufweist, die zu der in SEQ ID NO:4 angegebenen Aminosäuresequenz über deren Gesamtlänge zu mindestens 80%, bevorzugt zu mindestens 90 %, insbesondere zu 100 % identisch ist und die optional mindestens eine Aminosäuresubstitution an einer, zwei, drei oder vier der folgenden Positionen 3, 4, 99 und 199 in der Zählung gemäß SEQ ID NO:4 aufweist. Bevorzugt werden Proteasen eingesetzt, die an zwei, bevorzugt drei oder mehr, insbesondere vier der vorstehend genannten Positionen eine Aminosäuresubstitution aufweisen.

Weitere einsetzbare Varianten sind solche, die eine Aminosäuresequenz besitzen, die zu der in SEQ ID NO:4 angegebenen Aminosäuresequenz mindestens zu 81%, 82%, 83%, 84%, 85%, 86%, 87%, 88%, 89%, 90%, 91%, 92%, 93%, 94%, 95%, 96%, 97%, 98% oder 99% identisch ist, und die mindestens eine Aminosäuresubstitution an einer der folgenden Positionen 3, 4, 99 und 199 aufweisen.

Besonders bevorzugt weist eine derartige Protease eine Aminosäuresequenz auf, die zu der in SEQ ID NO:4 angegebenen Aminosäuresequenz über deren Gesamtlänge zu mindestens 80% identisch ist und die in der Zählung gemäß SEQ ID NO:4 die Aminosäuresubstitution R99E oder R99D, sowie optional zusätzlich mindestens eine oder zwei, vorzugsweise alle drei der Aminosäuresubstitutionen S3T, V4I und V199I.

Bevorzugt weist eine derartige Protease eine Aminosäuresequenz auf, die in der Zählung gemäß SEQ ID NO:4 mindestens eine, bevorzugt mehrere, insbesondere jede der folgenden Aminosäuresubstitution R99E/R99D, S3T, V4I und/oder V199I aufweist und an allen anderen Stellen zu der in SEQ ID NO:4 angegebenen Aminosäuresequenz über deren Gesamtlänge zu mindestens 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% insbesondere zu 100 % identisch ist. Besonders bevorzugt ist eine Protease, die eine Aminosäuresequenz aufweist, die ausgehend von der Aminosäuresequenz mit der SEQ ID NO:4 durch eine oder mehrere der Aminosäuresubstitutionen R99E/R99D, S3T, V4I und V199I gemäß der Zählung nach SEQ ID NO:4 erhältlich ist. Eine derartige Protease kann die in einer der SEQ ID Nos:7-8 angegebene Aminosäuresequenz aufweisen.

Es ist erfindungsgemäß besonders bevorzugt, eine Kombination einer ersten und einer zweiten Protease einzusetzen. Als erste Protease wird dabei insbesondere eine Variante der Protease mit der Aminosäuresequenz gemäß SEQ ID NO:3 eingesetzt, vorzugsweise eine wie oben beschriebene Variante, die mindestens eine Aminosäuresubstitution an einer der Positionen 9, 15, 66, 212 und 239 in der Zählung gemäß SEQ ID NO:3 aufweist. In einer Ausführungsform ist diese erste Protease zu der in SEQ ID NO:3 angegebenen Aminosäuresequenz über deren Gesamtlänge zu mindestens 80 %, 81%, 82%, 83%, 84%, 85%, 86%, 87%, 88%, 89%, 90%, 91%, 92%, 93%, 94%, 95%, 96%, 97%, 98% oder 99% identisch ist und umfasst vier oder ganz besonders bevorzugt 5 der Aminosäuresubstitutionen ausgewählt aus S9R, A15T, V66A, N212D und Q239R bezogen auf die Zählung nach SEQ ID NO:3. Ganz besonders bevorzugt ist die erste Protease eine Protease mit der Aminosäuresequenz nach SEQ ID NO:5.

Die zweite Protease wird aus den übrigen oben beschriebenen ausgewählt. Bevorzugte Kombinationen sind solche der Protease mit der SEQ ID NO:5 mit einer der Proteasen mit den Aminosäuresequenzen gemäß SEQ ID Nos. 6-8.

In verschiedenen Ausführungsformen werden Kombinationen von zwei Proteasen in einem Massenverhältnis jeweils bezogen auf Aktivprotein von 10:1 bis 1:10, vorzugsweise 5:1 bis 1:5, insbesondere von 3:1 bis 1:1, beispielsweise 2:1eingesetzt.

Überraschenderweise zeigen die hierin beschriebenen Kombinationen von verschiedenen Amylasen und mindestens einer Protease die Eigenschaft, die Leistung der Reinigungsmittels, bevorzugt des Geschirrspülmittels zu verbessern, indem sie zu einer verbesserten Reinigungsleistung an hartnäckigen, beispielsweise angebrannten, Anschmutzungen führen. Die Steigerung der Reinigungsleistung ist auch bei niedrigen Temperaturen, d.h. Temperaturen, die niedriger sind als die üblicherweise in Geschirrspülverfahren verwendeten, wie oben definiert, zu beobachten. Das ermöglicht es, das Reinigungsverfahren, bevorzugt das automatische Geschirrspülverfahren bei niedrigeren Temperaturen durchzuführen und trotzdem eine gute Reinigungsleistung beizubehalten.

Dabei ist in der Regel unter der Verbesserung der Reinigungsleistung zu verstehen, dass bei Verwendung der hierin beschriebenen Reinigungsmittel, insbesondere der Geschirrspülmittel die Entfernung von Anschmutzungen, insbesondere angebrannten Anschmutzungen, auf harten Oberflächen, insbesondere Geschirr, bei dessen Reinigung bevorzugt in einer automatischen Geschirrspülmaschine im Vergleich zu der Verwendung von Reinigungsmitteln, bevorzugt Geschirrspülmitteln, die die hierin beschriebenen Enzymkombinationen nicht enthalten, merklich verbessert ist.

Die einzusetzenden Enzyme können ferner zusammen mit Begleitstoffen, etwa aus der Fermentation, oder mit Stabilisatoren konfektioniert sein.

Die Reinigungsmittel, insbesondere Geschirrspülmittel, enthalten jede Amylase und ggf. auch jede Protease vorzugsweise jeweils in einer Menge des jeweiligen aktiven Proteins von 1 x 10⁻⁸ bis 5 Gew.-% bezogen auf das Gesamtgewicht des Mittels. Bevorzugt sind die Enzyme jeweils von 0,001 bis 2 Gew.-%, weiter bevorzugt von 0,005 bis 1,5 Gew.-%, noch weiter bevorzugt von 0,0075 bis 1 Gew.-% und besonders bevorzugt von 0,01 bis 0,6 Gew.-% Aktivprotein in diesen Mitteln enthalten.

In besonders bevorzugten Ausführungsformen wird in den hierin beschriebenen Mitteln die erste Amylase in einer Gesamtmenge des aktiven Proteins bezogen auf das Gesamtgewicht des Reinigungsmittels vom 0,01 bis 1 Gew.-%, vorzugsweise 0,025 bis 0,6 Gew.-% eingesetzt. In gleicher Weise wird vorzugsweise die zweite Amylase in einer Gesamtmenge des aktiven Proteins von 0,005 bis 0,6 Gew.-%, vorzugsweise 0,0075 bis 0,2 Gew.-% eingesetzt.

Als erste Amylase werden bevorzugt 0,01 bis 1 Gew.-%, vorzugsweise 0,025 bis 0,6 Gew.-% einer Amylase eingesetzt, die eine Aminosäuresequenz umfasst, die zu der in SEQ ID NO:1 angegebenen Aminosäuresequenz über deren Gesamtlänge zu mindestens 80% identisch ist und optional mindestens eine Aminosäuresubstitution an einer der Positionen 172, 202, 208, 255 und 261 in der Zählung gemäß SEQ ID NO:1 aufweist, insbesondere ausgewählt aus der Gruppe bestehend aus M202L, M202V, M202S, M202T, M202I, M202Q, M202W, S255N und R172Q, und als zweite Amylase 0,005 bis 0,6 Gew.-%, vorzugsweise 0,0075 bis 0,2 Gew.-% einer Amylase, die eine Aminosäuresequenz, die zu der in SEQ ID NO:2 angegebenen Aminosäuresequenz über deren Gesamtlänge zu mindestens 80% identisch ist und in der Zählung gemäß SEQ ID NO:2 Aminosäuresubstitutionen an drei oder mehr der Positionen 9, 26, 149, 182, 186, 202, 257, 295, 299, 323, 339 and 345 und optional eine oder mehrere, vorzugsweise alle, der Substitutionen und/oder Deletionen an den Positionen: 118, 183, 184, 195, 320 und 458, besonders bevorzugt R118K, D183*, G184*, N195F, R320K und/oder R458K aufweist und an allen anderen Stellen zu der in SEQ ID NO:2 angegebenen Aminosäuresequenz über deren Gesamtlänge zu 100 % identisch ist.

In der vorstehend beschriebenen Kombination aus erster und zweiter Amylase wird ferner mindestens eine Protease, noch bevorzugter eine erste und eine zweite Protease eingesetzt. Als erste Protease wird dabei insbesondere eine Variante der Protease mit der Aminosäuresequenz gemäß SEQ ID NO:3 eingesetzt, vorzugsweise eine wie oben beschriebene Variante, die mindestens eine Aminosäuresubstitution an einer der Positionen 9, 15, 66, 212 und 239 in der Zählung gemäß SEQ ID NO:3 aufweist. In einer Ausführungsform ist diese erste Protease zu der in SEQ ID NO:3 angegebenen Aminosäuresequenz über deren Gesamtlänge zu mindestens 80 %, 81%, 82%, 83%, 84%, 85%, 86%, 87%, 88%, 89%, 90%, 91%, 92%, 93%, 94%, 95%, 96%, 97%, 98% oder 99% identisch ist und umfasst vier oder ganz besonders bevorzugt 5 der Aminosäuresubstitutionen ausgewählt aus S9R, A15T, V66A, N212D und Q239R bezogen auf die Zählung nach SEQ ID NO:3. Ganz besonders bevorzugt ist die erste Protease eine Protease mit der Aminosäuresequenz nach SEQ ID NO:5.

Sofern noch eine zweite Protease enthalten ist, wird diese vorzugsweise aus den Proteasen mit den in SEQ ID Nos. 6-8 angegebenen Aminosäuresequenzen ausgewählt.

In flüssigen Formulierungen werden die Enzyme bevorzugt als Enzymflüssigformulierung(en) eingesetzt.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren bestimmt werden. Die Bestimmung der Aktivproteinkonzentration erfolgt diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913).

Enzyme, insbesondere die hierin beschriebenen Amylasen und Proteasen, können besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung ist eine Inhibierung der Proteolyse besonders bevorzugt. Die beschriebenen Mittel können zu diesem Zweck Stabilisatoren enthalten.

Reinigungsaktive Enzyme werden in der Regel nicht in Form des reinen Proteins sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Wie aus der vorherigen Ausführungen ersichtlich, bildet das Enzym-Protein nur einen Bruchteil des Gesamtgewichts üblicher Enzym-Zubereitungen. Bevorzugt eingesetzte Enzym-Zubereitungen enthalten zwischen 0,1 und 40 Gew.-%, bevorzugt zwischen 0,2 und 30 Gew.-%, besonders bevorzugt zwischen 0,4 und 20 Gew.-% und insbesondere zwischen 0,8 und 15 Gew.-% des Enzymproteins. Die beschriebenen Mittel enthalten derartige Enzym-Zubereitungen daher vorzugsweise in einer Menge von jeweils 0,1 bis 10 Gew.-%, vorzugsweise 0,2 bis 5 Gew.-% bezogen auf das Gesamtmittel.

Die hierin beschriebenen Reinigungsmittel, insbesondere die bevorzugten maschinellen Geschirrspülmittel können fester oder flüssiger Natur sein und insbesondere als pulverförmige Feststoffe, in nachverdichteter Teilchenform, als homogene Lösungen oder Suspensionen vorliegen. In einer weiteren bevorzugten Ausführungsform der Erfindung liegt das maschinelle Geschirrspülmittel in einer vorportionierten Form vor. In einer weiteren bevorzugten Ausführungsform der Erfindung weist das maschinelle Geschirrspülmittel mehrere räumlich voneinander getrennte Zusammensetzungen auf, wodurch es möglich ist, nicht kompatible Inhaltsstoffe voneinander zu trennen, oder Zusammensetzungen in Kombination anzubieten, welche zu unterschiedlichen Zeitpunkten in der Geschirrspülmaschine zum Einsatz kommen. Dies ist besonders vorteilhaft, wenn die maschinellen Geschirrspülmittel in vorportionierter Form vorliegen. Dabei kann mindestens eine der Zusammensetzungen fest und/oder mindestens eine der Zusammensetzungen flüssig vorliegen, wobei die Amylasen und optional auch Proteasen in mindestens einer der Zusammensetzungen enthalten sind, aber auch in mehreren Zusammensetzungen vorliegen können.

Vorzugsweise enthalten die hierin beschriebenen Mittel mindestens einen weiteren Bestandteil, insbesondere mindestens zwei weitere Bestandteile, ausgewählt aus der Gruppe bestehend aus Gerüststoffen, Tensiden, Polymeren, Bleichmitteln, Bleichkatalysatoren, Bleichaktivatoren, Nicht-Protease- und Nicht-Amylase-Enzymen, Korrosionsinhibitoren und Glaskorrosionsinhibitoren, Desintegrationshilfsmitteln, Duftstoffen und Parfümträgern.

Nachfolgend werden mögliche Inhaltsstoffe beschrieben, welche vorteilhafterweise in den hierin beschriebenen Reinigungsmittel, insbesondere Geschirrspülmittel, bevorzugt maschinellen Geschirrspülmitteln eingesetzt werden können.

Vorteilhafterweise können Gerüststoffe eingesetzt werden. Zu den Gerüststoffen zählen insbesondere die Zeolithe, Silikate, Carbonate, organische Cobuilder und - wo keine ökologischen Vorurteile gegen ihren Einsatz bestehen - auch die Phosphate.

In den hierin beschriebenen Mitteln können kristalline schichtförmige Silikate eingesetzt werden. Solche Reinigungsmittel, bevorzugt Geschirrspülmittel, insbesondere maschinellen Geschirrspülmittel enthalten vorzugsweise einen Gewichtsanteil an kristallinem schichtförmigen Silikat von 0,1 bis 20 Gew.-%, bevorzugt von 0,2 bis 15 Gew.-% und insbesondere von 0,4 bis 10 Gew.-%, jeweils bezogen auf das Gesamtgewicht dieser Mittel.

Auch ein Einsatz der allgemein bekannten Phosphate als Buildersubstanzen ist möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- bzw. Pentakaliumtriphosphat (Natrium- bzw. Kaliumtripolyphosphat) in der Wasch- oder Reinigungsmittel-Industrie die größte Bedeutung.

Alkalimetallphosphate ist dabei die summarische Bezeichnung für die Alkalimetall- (insbesondere Natrium- und Kalium-) Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen bzw. Kalkinkrustationen in Geweben und tragen überdies zur Reinigungsleistung bei.

Technisch besonders wichtige Phosphate sind das Pentanatriumtriphosphat, Na₅P₃O₁₀ (Natriumtripolyphosphat) sowie das entsprechende Kaliumsalz Pentakaliumtriphosphat, K₅P₃O₁₀ (Kaliumtripolyphosphat). Bevorzugt einsetzbar sind die Natriumkaliumtripolyphosphate.

Werden im Rahmen der vorliegenden Anmeldung Phosphate als wasch- oder reinigungsaktive Substanzen in den Reinigungsmitteln, bevorzugt Geschirrspülmitteln, insbesondere in maschinellen Geschirrspülmittel eingesetzt, so enthalten bevorzugte Mittel diese(s) Phosphat(e), vorzugsweise Alkalimetallphosphat(e), besonders bevorzugt Pentanatrium- bzw. Pentakaliumtriphosphat (Natrium- bzw. Kaliumtripolyphosphat), in Mengen von 5 bis 80 Gew.-%, vorzugsweise von 15 bis 75 Gew.-% und insbesondere von 20 bis 70 Gew.-%, jeweils bezogen auf das Gewicht des Reinigungsmittel, bevorzugt Geschirrspülmittels, insbesondere maschinellen Geschirrspülmittels. Es ist allerdings bevorzugt, dass die hierin beschriebenen Reinigungsmittel im Wesentlichen frei von Phosphaten, insbesondere den vorgenannten Phosphaten sind.

"Phosphatfrei", wie hierin verwendet, bedeutet, dass die betreffende Zusammensetzung im Wesentlichen frei von Phosphaten ist, d.h. insbesondere Phosphate in Mengen kleiner als 0,1 Gew.-%, vorzugsweise kleiner als 0,01 Gew.-% bezogen auf die Gesamtzusammensetzung enthält. Sofern doch Phosphate enthalten sind, werden diese vorzugsweise in Mengen eingesetzt, die nicht mehr als 0,3g/job entsprechen. Der Ausdruck g pro job (g/job) oder g/Anwendung bedeutet die eingesetzte Menge des Aktivstoffes im Verhältnis zum Gesamtgewicht des für einen vollständigem Reinigungszyklus eingesetzten Mittels (also für maschinelle Geschirrspülmittel die in einem vollständigen Reinigungsprogramm einer Geschirrspülmaschine eingesetzte Gesamtmenge des Reinigungsmittels. Bei vorportionierten Reinigungsmitteln (bevorzugt maschinellen Geschirrspülmitteln) ist diese Angabe die Menge des Aktivstoffes in g bezogen auf das Gesamtgewicht des vorproportionierten Reinigungsmittels. Der Ausdruck "Phosphate", wie in diesem Zusammenhang verwendet, schließt nicht die unten beschriebenen Phosphonate ein.

Weitere Gerüststoffe sind die Alkaliträger. Als Alkaliträger gelten beispielsweise Alkalimetallhydroxide, Alkalimetallcarbonate, Alkalimetallhydrogencarbonate, Alkalimetallsesquicarbonate, die genannten Alkalisilikate, Alkalimetasilikate, und Mischungen der vorgenannten Stoffe, wobei im Sinne dieser Erfindung bevorzugt die Alkalicarbonate, insbesondere Natriumcarbonat, Natriumhydrogencarbonat oder Natriumsesquicarbonat eingesetzt werden können. Besonders bevorzugt ist ein Buildersystem enthaltend eine Mischung aus Tripolyphosphat und Natriumcarbonat. Ebenfalls besonders bevorzugt ist ein Buildersystem enthaltend eine Mischung aus Tripolyphosphat und Natriumcarbonat und Natriumdisilikat. Aufgrund ihrer im Vergleich mit anderen Buildersubstanzen geringen chemischen Kompatibilität mit den übrigen Inhaltsstoffen von Reinigungsmitteln, insbesondere Geschirrspülmitteln, bevorzugt maschinellen Geschirrspülmitteln, werden die optionalen Alkalimetallhydroxide bevorzugt nur in geringen Mengen oder überhaupt nicht eingesetzt.

Besonders bevorzugt ist der Einsatz von Carbonat(en) und/oder Hydrogencarbonat(en), vorzugsweise Alkalicarbonat(en), besonders bevorzugt Natriumcarbonat, in Mengen von 2 bis 50 Gew.-%, vorzugsweise von 5 bis 40 Gew.-% und insbesondere von 7,5 bis 30 Gew.-%, jeweils bezogen auf das Gewicht des Mittels, bevorzugt maschinellen Geschirrspülmittels. Besonders bevorzugt werden Mittel, welche bezogen auf das Gewicht des maschinellen Geschirrspülmittels weniger als 20 Gew.-%, vorzugsweise weniger als 17 Gew.-%, bevorzugt weniger als 13 Gew.-% und insbesondere weniger als 9 Gew.-% Carbonat(e) und/oder Hydrogencarbonat(e), vorzugsweise Alkalicarbonat(e), besonders bevorzugt Natriumcarbonat enthalten.

Als organische Cobuilder sind insbesondere Polycarboxylate / Polycarbonsäuren, polymere Polycarboxylate, Asparaginsäure, Polyacetale, Dextrine, weitere organische Cobuilder sowie Phosphonate zu nennen. Diese Stoffklassen werden nachfolgend beschrieben.

Brauchbare organische Gerüstsubstanzen sind beispielsweise die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Nitrilotriessigsäure (NTA), sofern ein derartiger Einsatz aus ökologischen Gründen nicht zu beanstanden ist, sowie Mischungen aus diesen. Die freien Säuren besitzen neben ihrer Builderwirkung typischerweise auch die Eigenschaft einer Säuerungskomponente und dienen somit auch zur Einstellung eines niedrigeren und milderen pH-Wertes Mittels. Insbesondere sind hierbei Citronensäure, Bernsteinsäure, Glutarsäure, Adipinsäure, Gluconsäure und beliebige Mischungen aus diesen zu nennen.

Als besonders vorteilhaft für die Reinigungs- und Klarspülleistung hierin beschriebener Mittel hat sich der Einsatz von Citronensäure und/oder Citraten in diesen Mitteln erwiesen. Bevorzugt werden daher Reinigungsmittel, bevorzugt Geschirrspülmittel, insbesondere bevorzugt maschinelle Geschirrspülmittel, dadurch gekennzeichnet, dass das Mittel Citronensäure oder ein Salz der Citronensäure enthält und das der Gewichtsanteil der Citronensäure oder des Salzes der Citronensäure vorzugsweise mehr als 10 Gew.-%, bevorzugt mehr als 15 Gew.-% und insbesondere zwischen 20 und 40 Gew.-% beträgt.

Eine weitere bedeutende Klasse der Gerüststoffe stellen Aminocarbonsäuren und/oder ihre Salze dar. Besonders bevorzugte Vertreter dieser Klasse sind Methylglycindiessigsäure (MGDA) oder ihre Salze sowie Glutamindiessigsäure (GLDA) oder ihre Salze oder Ethylendiamindiessigsäure oder ihre Salze (EDDS). Ebenfalls geeignet sind Iminodibernsteinsäure (IDS) und Iminodiessigsäure (IDA). Der Gehalt an diesen Aminocarbonsäuren bzw. ihren Salzen kann beispielsweise zwischen 0,1 und 15 Gew.-% bevorzugt zwischen 0,5 und 10 Gew.-% und insbesondere zwischen 0,5 und 6 Gew.-% ausmachen. Aminocarbonsäuren und ihre Salze können zusammen mit den vorgenannten Gerüststoffen, insbesondere auch mit den phosphatfreien Gerüststoffen eingesetzt werden.

Erfindungsgemäß werden als Gerüststoffe vorzugsweise phosphatfreie Gerüststoffe, insbesondere ausgewählt aus Citrat, GLDA und/oder MGDA sowie ggf. EDDS und/oder IDS eingesetzt.

Als Gerüststoffe sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol sowie beispielsweise Polyaspartate und/oder Polyglutamate, jeweils entweder alpha-ständig oder seitenkettenverknüpft, oder deren Copolymere. Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure.

Die (co-)polymeren Polycarboxylate können entweder als Pulver oder als wässrige Lösung eingesetzt werden. Der Gehalt der Reinigungsmittel, bevorzugt Geschirrspülmittel, insbesondere maschinellen Geschirrspülmittel an (co-)polymeren Polycarboxylaten beträgt vorzugsweise 0,5 bis 20 Gew.-% und insbesondere 3 bis 10 Gew.-%.

Zur Verbesserung der Wasserlöslichkeit können die Polymere auch Allylsulfonsäuren, wie beispielsweise Allyloxybenzolsulfonsäure und Methallylsulfonsäure, als Monomer enthalten. Weitere bevorzugte Copolymere sind solche, die als Monomere Acrolein und Acrylsäure/Acrylsäuresalze bzw. Acrolein und Vinylacetat aufweisen.

Die Reinigungsmittel können als Gerüststoff insbesondere auch Phosphonate enthalten. Als Phosphonat-Verbindung wird vorzugsweise ein Hydroxyalkan- und/oder Aminoalkanphosphonat eingesetzt. Unter den Hydroxyalkanphosphonaten ist das 1-Hydroxyethan-1,1-diphosphonat (HEDP) von besonderer Bedeutung. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Phosphonate sind in den Mitteln vorzugsweise in Mengen von 0,1 bis 10 Gew.-%, insbesondere in Mengen von 0,5 bis 8 Gew.-%, jeweils bezogen auf das Gesamtgewicht des Reinigungsmittels, enthalten.

Darüber hinaus können alle Verbindungen, die in der Lage sind, Komplexe mit Erdalkaliionen auszubilden, als Gerüststoffe eingesetzt werden.

Die hierin beschriebenen Mittel können Tenside enthalten, wobei zur Gruppe der Tenside die nichtionischen, die anionischen, die kationischen und die amphoteren Tenside gezählt werden.

Als nichtionische Tenside können alle dem Fachmann bekannten nichtionischen Tenside eingesetzt werden. Als nichtionische Tenside eignen sich beispielsweise Alkylglykoside der allgemeinen Formel RO(G)ₓ, in der R einem primären geradkettigen oder methylverzweigten, insbesondere in 2-Stellung methylverzweigten aliphatischen Rest mit 8 bis 22, vorzugsweise 12 bis 18 C-Atomen entspricht und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse bevorzugt einsetzbarer nichtionischer Tenside, die entweder als alleiniges nichtionisches Tensid oder in Kombination mit anderen nichtionischen Tensiden eingesetzt werden können, sind alkoxylierte, vorzugsweise ethoxylierte oder ethoxylierte und propoxylierte Fettsäurealkylester, vorzugsweise mit 1 bis 4 Kohlenstoffatomen in der Alkylkette.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind die als PHFA bekannten Polyhydroxyfettsäureamide.

Als bevorzugte Tenside können schwachschäumende nichtionische Tenside eingesetzt werden. Mit besonderem Vorzug enthalten die Reinigungsmittel, bevorzugt Geschirrspülmittel, insbesondere maschinellen Geschirrspülmittel nichtionische Tenside aus der Gruppe der alkoxylierten Alkohole. Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 Mol EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt einer ganzen oder einer gebrochenen Zahl entsprechen können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Insbesondere bevorzugt sind nichtionische Tenside, die einen Schmelzpunkt oberhalb Raumtemperatur aufweisen. Nichtionische(s) Tensid(e) mit einem Schmelzpunkt oberhalb von 20°C, vorzugsweise oberhalb von 25°C, besonders bevorzugt zwischen 25 und 60°C und insbesondere zwischen 26,6 und 43,3°C, ist/sind besonders bevorzugt.

Bevorzugt einzusetzende Tenside stammen aus den Gruppen der alkoxylierten Niotenside, insbesondere der ethoxylierten primären Alkohole.

Aniontenside können ebenfalls als Bestandteil von Reinigungsmitteln, bevorzugt Geschirrspülmitteln, insbesondere von maschinellen Geschirrspülmitteln eingesetzt werden. Zu ihnen zählen insbesondere Alkylbenzolsulfonate, (Fett-)Alkylsulfate, (Fett-)Alkylethersulfate sowie Alkansulfonate. Der Gehalt der Mittel an Aniontensiden beträgt üblicherweise 0 bis 10 Gew.-%.

An Stelle der genannten Tenside oder in Verbindung mit ihnen können auch kationische und/oder amphotere Tenside eingesetzt werden. In Reinigungsmittel, insbesondere Geschirrspülmitteln, bevorzugt maschinellen Geschirrspülmitteln, beträgt der Gehalt an kationischen und/oder amphoteren Tensiden vorzugsweise weniger als 6 Gew.-%, bevorzugt weniger als 4 Gew.-%, ganz besonders bevorzugt weniger als 2 Gew.-% und insbesondere weniger als 1 Gew.-%. Mittel, welche keine kationischen oder amphoteren Tenside enthalten, werden besonders bevorzugt.

Zur Gruppe der Polymere zählen insbesondere die wasch- oder reinigungsaktiven Polymere, beispielsweise die Klarspülpolymere und/oder als Enthärter wirksame Polymere. Generell sind in maschinellen Geschirrspülmitteln neben nichtionischen Polymeren auch kationische, anionische und amphotere Polymere einsetzbar.

"Kationische Polymere" im Sinne der vorliegenden Erfindung sind Polymere, welche eine positive Ladung im Polymermolekül tragen. Diese kann beispielsweise durch in der Polymerkette vorliegende (Alkyl-)Ammoniumgruppierungen oder andere positiv geladene Gruppen realisiert werden. Besonders bevorzugte kationische Polymere stammen aus den Gruppen der quaternierten Cellulose-Derivate, der Polysiloxane mit quaternären Gruppen, der kationischen Guar-Derivate, der polymeren Dimethyldiallylammoniumsalze und deren Copolymere mit Estern und Amiden von Acrylsäure und Methacrylsäure, der Copolymere des Vinylpyrrolidons mit quaternierten Derivaten des Dialkylaminoacrylats und -methacrylats, der Vinylpyrrolidon-Methoimidazoliniumchlorid-Copolymere, der quaternierter Polyvinylalkohole oder der unter den INCI-Bezeichnungen Polyquaternium 2, Polyquaternium 17, Polyquaternium 18 und Polyquaternium 27 angegeben Polymere.

"Amphotere Polymere" im Sinne der vorliegenden Erfindung weisen neben einer positiv geladenen Gruppe in der Polymerkette weiterhin auch negativ geladenen Gruppen bzw. Monomereinheiten auf. Bei diesen Gruppen kann es sich z.B. um Carbonsäuren, Sulfonsäuren oder Phosphonsäuren handeln.

Bevorzugte einsetzbare amphotere Polymere stammen aus der Gruppe der Alkylacrylamid/Acrylsäure-Copolymere, der Alkylacrylamid/Methacrylsäure-Copolymere, der Alkylacrylamid/Methylmethacrylsäure-Copolymere, der Alkylacrylamid/Acrylsäure/Alkylaminoalkyl(meth)acrylsäure-Copolymere, der Alkylacrylamid/Methacrylsäure/Alkylaminoalkyl(meth)-acrylsäure-Copolymere, der Alkylacrylamid/Methylmethacrylsäure/Alkylaminoalkyl(meth)acrylsäure-Copolymere, der Alkylacrylamid/Alkymethacrylat/Alkylaminoethylmethacrylat/Alkylmethacrylat-Copolymere sowie der Copolymere aus ungesättigten Carbonsäuren, kationisch derivatisierten ungesättigten Carbonsäuren und gegebenenfalls weiteren ionischen oder nichtionogenen Monomeren.

Bevorzugt einsetzbare zwitterionische Polymere stammen aus der Gruppe der Acrylamidoalkyltrialkylammoniumchlorid/Acrylsäure-Copolymere sowie deren Alkali- und Ammoniumsalze, der Acrylamidoalkyltrialkylammoniumchlorid/Methacrylsäure-Copolymere sowie deren Alkali- und Ammoniumsalze und der Methacroylethylbetain/Methacrylat-Copolymere.

Reinigungsmittel, insbesondere Geschirrspülmittel, bevorzugt maschinelle Geschirrspülmittel enthalten die vorgenannten kationischen und/oder amphoteren Polymere vorzugsweise in Mengen zwischen 0,01 und 10 Gew.-%, jeweils bezogen auf das Gesamtgewicht des maschinellen Geschirrspülmittels. Bevorzugt werden im Rahmen der vorliegenden Anmeldung jedoch solche maschinelle Geschirrspülmittel, bei denen der Gewichtsanteil der kationischen und/oder amphoteren Polymere zwischen 0,01 und 8 Gew.-%, vorzugsweise zwischen 0,01 und 6 Gew.-%, bevorzugt zwischen 0,01 und 4 Gew.-%, besonders bevorzugt zwischen 0,01 und 2 Gew.-% und insbesondere zwischen 0,01 und 1 Gew.-%, jeweils bezogen auf das Gesamtgewicht des maschinellen Geschirrspülmittels, beträgt.

In den Reinigungsmitteln, bevorzugt Geschirrspülmitteln, insbesondere maschinellen Geschirrspülmitteln können ferner Bleichmittel eingesetzt werden. Unter den als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure. Einsetzbar sind außerdem alle weiteren dem Fachmann aus dem Stand der Technik bekannten anorganischen oder organischen Peroxybleichmittel.

Als Bleichmittel können auch Chlor oder Brom freisetzende Substanzen eingesetzt werden. Unter den geeigneten Chlor oder Brom freisetzenden Materialien kommen beispielsweise heterozyklische N-Brom- und N-Chloramide, beispielsweise Trichlorisocyanursäure, Tribromisocyanursäure, Dibromisocyanursäure und/oder Dichlorisocyanursäure (DICA) und/oder deren Salze mit Kationen wie Kalium und Natrium in Betracht. Hydantoinverbindungen, wie 1,3-Dichlor-5,5-dimethylhydanthoin sind ebenfalls geeignet.

Es werden Reinigungsmittel, bevorzugt Geschirrspülmittel, insbesondere maschinelle Geschirrspülmittel bevorzugt, die 1 bis 35 Gew.-%, vorzugsweise 2,5 bis 30 Gew.-%, besonders bevorzugt 3,5 bis 20 Gew.-% und insbesondere 5 bis 15 Gew.-% Bleichmittel, vorzugsweise Natriumpercarbonat, enthalten.

Ferner können die Reinigungsmittel, bevorzugt Geschirrspülmittel, insbesondere maschinellen Geschirrspülmittel Bleichkatalysatoren enthalten. Die einsetzbaren Bleichkatalysatoren schließen ein, sind aber nicht beschränkt auf die Gruppe der bleichverstärkenden Übergangsmetallsalze und Übergangsmetallkomplexe, vorzugsweise der Mn-, Fe-, Co-, Ru - oder Mo-Komplexe, besonders bevorzugt aus der Gruppe der Mangan und/oder Cobaltsalze und/oder -komplexe, insbesondere der Cobalt(ammin)-Komplexe, der Cobalt(acetat)-Komplexe, der Cobalt(Carbonyl)-Komplexe, der Chloride des Cobalts oder Mangans, des Mangansulfats und der Komplexe des Mangans mit 1,4,7-trimethyl-1,4,7-triazacyclononan (Mn₃-TACN) oder 1,2,4,7-tetramethyl-1,4,7-tri-azacyclononan (Mn₄-TACN).

Es werden Reinigungsmittel, bevorzugt Geschirrspülmittel, insbesondere maschinelle Geschirrspülmittel bevorzugt, die 0,001 bis 1 Gew.-%, vorzugsweise 0,01 bis 0,1 Gew.-% Bleichkatalysator, vorzugsweise einen Mn-Komplex, insbesondere einen Komplex des Mangans mit 1,4,7-trimethyl-1,4,7-triazacyclononan (Mn₃-TACN) oder 1,2,4,7-tetramethyl-1,4,7-tri-azacyclononan (Mn₄-TACN), enthalten.

In verschiedenen Ausführungsformen der Erfindung enthalten die Reinigungsmittel, bevorzugt Geschirrspülmittel, insbesondere maschinellen Geschirrspülmittel zusätzlich mindestens einen Bleichaktivator. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Von allen dem Fachmann aus dem Stand der Technik bekannten Bleichaktivatoren werden mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS) besonders bevorzugt eingesetzt. Auch Kombinationen konventioneller Bleichaktivatoren können eingesetzt werden. Diese Bleichaktivatoren werden vorzugsweise in Mengen bis 10 Gew.-%, insbesondere 0,1 Gew.-% bis 8 Gew.-%, besonders 2 bis 8 Gew.-% und besonders bevorzugt 2 bis 6 Gew.-%, jeweils bezogen auf das Gesamtgewicht der bleichaktivatorhaltigen Mittel, eingesetzt.

Vorzugsweise enthalten die Mittel der vorliegenden Erfindung mindestens eine zusätzliche Enzymzubereitung oder Enzymzusammensetzung, die ein oder mehrere Nicht-Amylase- und Nicht-Protease-Enzyme enthält. Solche Enzyme umfassen, ohne darauf beschränkt zu sein, Lipasen, Cellulasen, Hemicellulasen, Mannanasen, Pektin-spaltende Enzyme, Tannasen, Xylanasen, Xanthanasen, β-Glucosidasen, Carrageenasen, Perhydrolasen, Oxidasen, Oxidoreduktasen sowie deren Gemische. Bevorzugte Enzyme umfassen insbesondere Cellulasen, Lipasen, Hemicellulasen, insbesondere Pectinasen, Mannanasen, β-Glucanasen, sowie deren Gemische. Besonders bevorzugt sind Lipasen. Diese Enzyme sind im Prinzip natürlichen Ursprungs; ausgehend von den natürlichen Molekülen stehen für den Einsatz in Wasch- oder Reinigungsmitteln verbesserte Varianten zur Verfügung, die entsprechend bevorzugt eingesetzt werden.

Die im Zusammenhang mit den eingesetzten Amylasen und Proteasen gemachten Angaben zu Mengen und Formulierungsformen treffen mutatis mutandis auch auf alle weiteren oben beschriebenen Enzyme zu.

Glaskorrosionsinhibitoren verhindern das Auftreten von Trübungen, Schlieren und Kratzern aber auch das Irisieren der Glasoberfläche von maschinell gereinigten Gläsern. Bevorzugte Glaskorro-sionsinhibitoren stammen aus der Gruppe der Magnesium- und Zinksalze sowie der Magnesium- und Zinkkomplexe. Im Rahmen der vorliegenden Erfindung beträgt der Gehalt an Zinksalz in Reinigungsmitteln, bevorzugt Geschirrspülmitteln, insbesondere maschinellen Geschirrspülmitteln vorzugsweise zwischen 0,1 bis 5 Gew.-%, bevorzugt zwischen 0,2 bis 4 Gew.-% und insbesondere zwischen 0,4 bis 3 Gew.-%, bzw. der Gehalt an Zink in oxidierter Form (berechnet als Zn²⁺) zwischen 0,01 bis 1 Gew.-%, vorzugsweise zwischen 0,02 bis 0,5 Gew.-% und insbesondere zwischen 0,04 bis 0,2 Gew.-%, jeweils bezogen auf das Gesamtgewicht des Glaskorrosionsinhibitor-haltigen Mittels.

Um den Zerfall vorgefertigter Formkörper zu erleichtern, ist es möglich, Desintegrationshilfsmittel, so genannte Tablettensprengmittel, in diese Mittel einzuarbeiten, um die Zerfallszeiten zu verkürzen. Unter Tablettensprengmitteln bzw. Zerfallsbeschleunigern werden Hilfsstoffe verstanden, die für den raschen Zerfall von Tabletten in Wasser oder anderen Medien und für die zügige Freisetzung der Wirkstoffe sorgen. Bevorzugt können Desintegrationshilfsmittel in Mengen von 0,5 bis 10 Gew.-%, vorzugsweise 3 bis 7 Gew.-% und insbesondere 4 bis 6 Gew.-%, jeweils bezogen auf das Gesamtgewicht des desintegrationshilfsmittelhaltigen Mittels, eingesetzt werden.

Als Parfümöle bzw. Duftstoffe können im Rahmen der vorliegenden Erfindung einzelne Riechstoffverbindungen, z.B. die synthetischen Produkte vom Typ der Ester, Ether, Aldehyde, Ketone, Alkohole und Kohlenwasserstoffe verwendet werden. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pinien-, Citrus-, Jasmin-, Patchouli-, Rosen- oder Ylang-Ylang-Öl.

Die Konfektionierung hierin beschriebener Reinigungsmittel, bevorzugt Geschirrspülmittel, insbesondere maschineller Geschirrspülmittel kann in unterschiedlicher Wiese erfolgen. Die Mittel können in fester oder flüssiger sowie als Kombination fester und flüssiger Angebotsformen vorliegen. Als feste Angebotsformen eignen sich insbesondere Pulver, Granulate, Extrudate, Kompaktate, insbesondere Tabletten. Die flüssigen Angebotsformen auf Basis von Wasser und/oder organischen Lösungsmitteln können verdickt, in Form von Gelen vorliegen. Hierin beschriebene Mittel können in Form einphasiger oder mehrphasiger Produkte konfektioniert werden.

Hierin beschriebene Reinigungsmittel, bevorzugt Geschirrspülmittel, insbesondere maschinelle Geschirrspülmittel werden vorzugsweise zu Dosiereinheiten vorkonfektioniert. Diese Dosiereinheiten umfassen vorzugsweise die für einen Reinigungsgang notwendige Menge an wasch- oder reinigungsaktiven Substanzen.

Die hierin beschriebenen Reinigungsmittel, bevorzugt Geschirrspülmittel, insbesondere maschinellen Geschirrspülmittel, insbesondere die vorgefertigten Dosiereinheiten weisen mit besonderem Vorzug eine wasserlösliche Umhüllung auf.

Die wasserlösliche Umhüllung wird vorzugsweise aus einem wasserlöslichen Folienmaterial, welches ausgewählt ist aus der Gruppe, bestehend aus Polymeren oder Polymergemischen, gebildet. Die Umhüllung kann aus einer oder aus zwei oder mehr Lagen aus dem wasserlöslichen Folienmaterial gebildet werden. Das wasserlösliche Folienmaterial der ersten Lage und der weiteren Lagen, falls vorhanden, kann gleich oder unterschiedlich sein. Besonders bevorzugt sind Folien, die beispielsweise zu Verpackungen wie Schläuchen oder Kissen verklebt und/oder versiegelt werden können, nachdem sie mit einem Mittel befüllt wurden.

Es ist bevorzugt, dass die wasserlösliche Umhüllung Polyvinylalkohol oder ein Polyvinylalkoholcopolymer enthält. Wasserlösliche Umhüllungen, die Polyvinylalkohol oder ein Polyvinylalkoholcopolymer enthalten, weisen eine gute Stabilität bei einer ausreichend hohen Wasserlöslichkeit, insbesondere Kaltwasserlöslichkeit, auf.

Besonders bevorzugt werden die vorgefertigten Dosiereinheiten durch eine wasserlösliche Umhüllung einer entsprechenden Portionsmenge an erfindungsgemäßer Zusammensetzung gebildet, die vorgefertigte Dosiereinheit umfasst also bevorzugt ein erfindungsgemäßes Mittel sowie eine wasserlösliche Umhüllung/Verpackung.

Bei den wasserlöslichen Umhüllungen/Verpackungen handelt es sich vorzugsweise um Tiefziehkörper oder Spritzgusskörper.

Die wasserlöslichen Behälter/Umhüllungen/Verpackungen können auch durch Spritzgießen hergestellt werden. Spritzgießen bezeichnet dabei das Umformen einer Formmasse derart, daß die in einem Massezylinder für mehr als einen Spritzgießvorgang enthaltene Masse unter Wärmeeinwirkung plastisch erweicht und unter Druck durch eine Düse in den Hohlraum eines vorher geschlossenen Werkzeuges einfließt. Das Verfahren wird hauptsächlich bei nichthärtbaren Formmassen angewendet, die im Werkzeug durch Abkühlen erstarren. Der Spritzguß ist ein sehr wirtschaftliches modernes Verfahren zur Herstellung spanlos geformter Gegenstände und eignet sich besonders für die automatisierte Massenfertigung. Im praktischen Betrieb erwärmt man die thermoplastische Formmassen (Pulver, Körner, Würfel, Pasten u. a.) bis zur Verflüssigung (bis 180 °C) und spritzt sie dann unter hohem Druck (bis 140 MPa) in geschlossene, zweiteilige, das heißt aus Gesenk (früher Matrize) und Kern (früher Patrize) bestehende, vorzugsweise wassergekühlte Hohlformen, wo sie abkühlen und erstarren. Einsetzbar sind Kolben- und Schneckenspritzgussmaschinen.

Solche Formkörper können ebenfalls ein, zwei, drei oder mehrere Kammern aufweisen und mit flüssigen und/oder festen Zusammensetzungen befüllt sein, von denen eine der Zusammensetzungen eine der erfindungsgemäßen Zusammensetzungen ist. Es ist beispielsweise möglich, die Kammern auf der offenen Seite entweder mit einem zweiten Formgußkörper oder mit einer oder mehreren wasserlöslichen Folien (insbesondere wie hierin beschrieben) zu verschließen. So kann die Freisetzung der in den Kammern befindlichen Zusammensetzungen beliebig nach dem gewünschten Freisetzungszeitpunkt gesteuert werden. Entweder kann das gesamte Mittel auf einmal (entweder direkt zu Beginn des Reinigungszyklus oder zu einem bestimmten Zeitpunkt im Verlauf des Reinigungszyklus) oder durch eine Variation der Folienzusammensetzung zu bestimmten, von einander verschiedenen Zeitpunkten innerhalb des Zyklus der Geschirrspülmaschine (beispielsweise in Abhängigkeit von der Temperatur des Spülwassers) freigesetzt werden.

Geeignete wasserlösliche Folien zur Herstellung der wasserlöslichen Umhüllung basieren bevorzugt auf einem Polyvinylalkohol oder einem Polyvinylalkoholcopolymer, dessen Molekulargewicht im Bereich von 10.000 bis 1.000.000 gmol⁻¹, vorzugsweise von 20.000 bis 500.000 gmol⁻¹, besonders bevorzugt von 30.000 bis 100.000 gmol⁻¹ und insbesondere von 40.000 bis 80.000 gmol⁻¹ liegt.

Die Herstellung von Polyvinylalkohol geschieht üblicherweise durch Hydrolyse von Polyvinylacetat, da der direkte Syntheseweg nicht möglich ist. Ähnliches gilt für Polyvinylalkoholcopolymere, die aus entsprechend aus Polyvinylacetatcopolymeren hergestellt werden. Bevorzugt ist, wenn wenigstens eine Lage der wasserlöslichen Umhüllung einen Polyvinylalkohol umfasst, dessen Hydrolysegrad 70 bis 100 Mol-%, vorzugsweise 80 bis 90 Mol-%, besonders bevorzugt 81 bis 89 Mol-% und insbesondere 82 bis 88 Mol-% ausmacht.

Einem zur Herstellung der wasserlöslichen Umhüllung geeignetem Polyvinylalkohol-enthaltendem Folienmaterial kann zusätzlich ein Polymer ausgewählt aus der Gruppe umfassend (Meth)Acrylsäure-haltige (Co)Polymere, Polyacrylamide, Oxazolin-Polymere, Polystyrolsulfonate, Polyurethane, Polyester, Polyether, Polymilchsäure oder Mischungen der vorstehenden Polymere zugesetzt sein. Ein bevorzugtes zusätzliches Polymer sind Polymilchsäuren.

Bevorzugte Polyvinylalkoholcopolymere umfassen neben Vinylalkohol Dicarbonsäuren als weitere Monomere. Geeignete Dicarbonsäuren sind Itaconsäure, Malonsäure, Bernsteinsäure und Mischungen daraus, wobei Itaconsäure bevorzugt ist.

Ebenfalls bevorzugte Polyvinylalkoholcopolymere umfassen neben Vinylalkohol eine ethylenisch ungesättige Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus.

Es kann bevorzugt sein, dass das Folienmaterial weitere Zusatzstoffe enthält. Das Folienmaterial kann beispielsweise Weichmacher wie Dipropylenglycol, Ethylenglycol, Diethylenglycol, Propylenglycol, Glycerin, Sorbitol, Mannitol oder Mischungen daraus enthalten. Weitere Zusatzstoffe umfassen beispielsweise Freisetzungshilfen, Füllmittel, Vernetzungsmittel, Tenside, Antioxidationsmittel, UV-Absorber, Antiblockmittel, Antiklebemittel oder Mischungen daraus.

Geeignete wasserlösliche Folien zum Einsatz in den wasserlöslichen Umhüllungen der wasserlöslichen Verpackungen gemäß der Erfindung sind Folien, die von der Firma MonoSol LLC beispielsweise unter der Bezeichnung M8630, C8400 oder M8900 vertrieben werden. Andere geeignete Folien umfassen Folien mit der Bezeichnung Solublon® PT, Solublon® GA, Solublon® KC oder Solublon® KL von der Aicello Chemical Europe GmbH oder die Folien VF-HP von Kuraray.

Die entsprechende Verwendung der hierin beschriebenen Mittel ist ebenfalls Gegenstand der Erfindung. Ebenso betrifft die Erfindung ein Geschirrspülverfahren, insbesondere maschinelles Geschirrspülverfahren, bei welchem ein Mittel gemäß der Erfindung eingesetzt wird. Gegenstand der vorliegenden Anmeldung ist daher weiterhin ein Verfahren zur Reinigung von Geschirr in einer Geschirrspülmaschine, bei welchem das Mittel während des Durchlaufens eines Geschirrspülprogramms vor Beginn des Hauptspülgangs oder im Verlaufe des Hauptspülgangs in den Innenraum einer Geschirrspülmaschine eindosiert wird. Die Eindosierung bzw. der Eintrag des Mittels in den Innenraum der Geschirrspülmaschine kann manuell erfolgen, vorzugsweise wird das Mittel jedoch mittels der Dosierkammer in den Innenraum der Geschirrspülmaschine dosiert. In verschiedenen Ausführungsformen der Erfindung liegt bei solchen Geschirrspülverfahren die (Spül-)Temperatur vorzugsweise bei 50 °C oder niedriger, besonders bevorzugt 45 °C oder niedriger, noch bevorzugter 40°C oder niedriger.

Schließlich betrifft die Erfindung auch die Verwendung der hierin beschriebenen Enzymkombinationen zur Verbesserung der Reinigungsleistung, insbesondere der Reinigungsleistung an enzymsensitiven Anschmutzungen, eines Reinigungsmittels, insbesondere eines Geschirrspülmittels, vorzugsweise eines maschinellen Geschirrspülmittels. Alle im Zusammenhang mit den hierin offenbarten Reinigungsmitteln als besondere Ausführungsformen beschriebenen Enzymkombinationen sind ebenso in den beschriebenen Verfahren und Verwendungen einsetzbar.

Eine typische Rahmenrezeptur für ein vorzugsweise einsetzbares maschinelles Geschirrspülmittel, beispielsweise in Tablettenform, umfasst folgende Stoffe:

| | |
|---|---|
| Na-Tripolyphosphat | 20-50 Gew.-% |
| Natriumcarbonat | 10-30 Gew.-% |
| Natriumpercarbonat | 5-18 Gew.-% |
| Bleichaktivator | 0,5-5 Gew.-% |
| Bleichkatalysator | 0,01-1 Gew.-% |
| Sulfopolymer | 2,5-15 Gew.-% |
| Polycarboxylat | 0,1-10 Gew.-% |
| Niotensid | 0,5-10 Gew.-% |
| Phosphonat | 0,5-5 Gew.-% |
| Proteasen | 0,1-5 Gew.-% |
| Amylasen | 0,1-5 Gew.-%, |

wobei sich die Angabe in Gew.-% jeweils auf das gesamte Mittel beziehen. Statt eines Teils des Tripolyphosphats kann in der Rezeptur insbesondere auch 10-50 Gew.-% Citrat oder MGDA oder GLDA oder EDDS oder Mischungen aus zwei oder drei dieser Substanzen eingesetzt werden.

### Beispiele

### Beispiel 1: Verbesserung der Amylaseleistung beim automatischen Geschirrspülen

**Tabelle 1: Zusammensetzung des maschinellen Geschirrspülmittels**

| | Basis |
|---|---|
| Tripolyphosphat (Gew.-%) | 35,9 |
| Natriumcarbonat (Gew.-%) | 12,2 |
| Phosphonat (Gew.-%) | 2,4 |
| Sulfonsäuregruppen-haltiges Polymer (Gew.-%) | 7,9 |
| Polyacrylat (Gew.-%) | 4,6 |
| Nichtionische Tenside (Gew.-%) | 6,1 |
| Percarbonat (Gew.-%) | 14,6 |
| TAED (Gew.-%) | 2,3 |
| Bleichkatalysator (Gew.-%) | 1,0 |
| Polycarboxylat (Gew.-%) | 1,5 |
| Natriumsilikat/Polycarboxylat (Gew.%) | 3,9 |
| Zinkacetat (Gew.-%) | 0,2 |
| Reste (Parfüm, Farbstoffe, Protease bzw. Proteasemischung etc.) (Gew.-%) | ad 100 |

### Reinigungsleistung an Spaghetti-Anschmutzungen

In einer Bosch Geschirrspülmaschine wurden bei 40 °C (Programm "Normal") und 21°dH Wasserhärte Porzellanteller mit einer Anschmutzung aus Spaghetti mit einer festen Geschirrspülmitteltablette (20 g; Zusammensetzung siehe Tabelle 1) mit zwei einzelnen Amylasen (Vergleichsversuche V1, V2) oder Amylasekombination (M1) gespült.

Nach jedem Spülzyklus wurde die Reinigungsleistung visuell nach IKW bestimmt (Auswertung von 1-10, je höher der Wert, desto besser die Leistung, Unterschiede von mindestens 1 sind signifikant). Die Ergebnisse für die gestesteten Rezepturen sind in der Tabelle 2 als arithmetische Mittelwerte von 3 Bestimmungen aufgelistet.

Zur Formulierung gemäß Tabelle 1 wurden (jeweils bezogen auf die Menge des Enzymproteins) entweder 50 mg/job Amylase 1 (V1) oder 5 mg/job bzw. Amylase 2 (V2) bzw. deren Mischung (M1, 25 mg/job Amylase 1 und 2,5 mg/job Amylase 2) zugegeben.

**Tabelle 2: Reinigungsleistung an Spaghetti**

| Amylase/Amylasekombination | Spaghetti |
|---|---|
| M1: | 3,8 |
| V1: | 2,8 |
| V2: | 2,2 |

| | |
|---|---|
| V1: Basis + 50mg/job 1. Amylase (Enzym gemäß Seq ID NO:1 mit den Aminosäuresubstitutionen M202L, S255N und R172Q) V2: Basis + 5 mg/job 2. Amylase bezogen auf Aktivprotein (Stainzyme Plus™) M1: Basis + Mischung der Enzyme gemäß V1 und V2 (25 mg/job Amylase 1 und 2,5 mg/job Amylase 2, jeweils bezogen auf die Menge an aktivem Protein). | |

Der Tabelle 2 ist eindeutig zu entnehmen, dass die Kombination der zwei verschiedenen Amylasen zu einer deutlichen Verbesserung der Reinigungsleistung führt (M1).

### Beispiel 2: Reinigungsleistung in phosphatfreien Geschirrspülmitteln

In einer Miele GSL Geschirrspülmaschine wurden bei 50 °C (Programm "Normal") und 21°dH und in einer Bosch SMS68M62 Geschirrspülmaschine bei 40 °C (Programm "Sanft 40") und 21°dH Porzellanteller mit einer Anschmutzung aus Tee (Assam, BOP, Lipton), Hackfleisch, Eigelb, Spaghetti, Stärke und Creme Brûlée mit einer festen Geschirrspülmitteltablette gespült. Zur Formulierung gemäß Tabelle 3 wurden verschiedene Enzyme in den angegebenen Mengen (mg Aktivprotein/job) zugegeben (V1-V6: Vergleichsversuche; E1-E3: erfindungsgemäß):

| | mg Protease 1 | mg Protease 2 | mg Amylase1 | mg Amylase2 |
|---|---|---|---|---|
| V1 | 50 | 25 | 0 | 5 |
| V2 | 80 | 0 | 0 | 5 |
| V3 | 0 | 80 | 0 | 5 |
| V4 | 80 | 0 | 50 | 0 |
| V5 | 0 | 80 | 50 | 0 |
| V6 | 50 | 25 | 50 | 0 |
| E1 | 80 | 0 | 25 | 2,5 |
| E2 | 50 | 25 | 25 | 2,5 |
| E3 | 0 | 80 | 25 | 2,5 |

| | | | | |
|---|---|---|---|---|
| Protease 1: Enzym gemäß SEQ ID NO:5 Protease 2: Enzym gemäß SEQ ID NO:6 Amylase 1: Enzym gemäß Seq ID NO:1 mit den Aminosäuresubstitutionen M202L, S255N und R172Q Amylase 2: Stainzyme Plus™ | | | | |

**Tabelle 3: Zusammensetzung des maschinellen Geschirrspülmittels in Gew.-% Aktivsubstanz**

| | Basis |
|---|---|
| Na-Citrat | 20 |
| MGDA | 16 |
| Phosphonat | 5 |
| Soda | 15 |
| Na-Percarbonat | 15 |
| Sulfonsäuregruppen-haltiges Polymer | 8 |
| Polyacrylat | 0,5 |
| Nichtionische Tenside | 7 |
| TAED | 3 |
| Bleichkatalysator | 1 |
| Zinkacetat | 0,2 |
| Na-sulfat | 0,5 |
| PEG 4000 | 0,5 |
| Reste (pH-Stellmittel, Parfüm, Farbstoffe etc.) | ad 100 |

Die Reinigungsleistung wurde visuell nach IKW bestimmt (Auswertung von 1-10, je höher der Wert, desto besser die Leistung, Unterschiede von mindestens 1 sind signifikant). Die Ergebnisse für die gestesteten Rezepturen sind in der Tabelle 4 als arithmetische Mittelwerte aufgelistet. Höhere Werte bedeuten eine bessere Reinigungsleistung.

**Tabelle 4: Summe der Reinigungsleistung von verschiedenen Anschmutzungen:**

| Enzymkombinationen (50°C) | |
|---|---|
| V1 | 65,1 |
| V2 | 62,3 |
| V3 | 61,3 |
| V4 | 66,2 |
| V5 | 66,2 |
| V6 | 67,1 |
| E1 | 68,6 |
| E2 | 70,5 |
| E3 | 68,5 |

Tabelle 4 ist deutlich zu entnehmen, dass die erfindungsgemäßen Kombinationen zu einer Erhöhung der Reinigungsleistung führt.

| Enzymkombinationen (40°C) | |
|---|---|
| V1 | 63,6 |
| V2 | 62,5 |
| V3 | 59 |
| V4 | 65 |
| V5 | 63,5 |
| V6 | 66,3 |
| E1 | 64,4 |
| E2 | 66,2 |
| E3 | 63,9 |

## Patentansprüche

1. Reinigungsmittel für harte Oberflächen, insbesondere ein Geschirrspülmittel, vorzugsweise ein maschinelles Geschirrspülmittel, **dadurch gekennzeichnet, dass**
- es mindestens eine erste und eine zweite Amylase umfasst, wobei
a) die erste Amylase eine α-Amylase aus Bacillus sp. No. 707 oder ein funktionales Fragment oder eine funktionale Variante davon ist,
b) die zweite Amylase eine AA560 α-Amylase aus Bacillus sp. oder ein funktionales Fragment oder eine funktionale Variante davon ist, und
- es ferner mindestens eine Protease enthält, wobei die mindestens eine Protease
(a) ein Subtilisin 309 aus *Bacillus lentus* oder ein funktionales Fragment oder eine funktionale Variante davon umfasst, insbesondere eine, die eine Aminosäuresequenz umfasst, die zu der in SEQ ID NO:3 angegebenen Aminosäuresequenz über deren Gesamtlänge zu mindestens 80%, bevorzugt zu mindestens 90%, insbesondere zu 100% identisch ist und mindestens eine Aminosäuresubstitution an einer der Positionen 9, 15, 66, 212 und 239 in der Zählung gemäß SEQ ID NO:3 aufweist, vorzugsweise ausgewählt aus der Gruppe bestehend aus S9R, A15T, V66A, N212D und Q239R; und/oder
(b) ein Subtilisin 309 aus *Bacillus lentus* oder ein funktionales Fragment oder eine funktionale Variante davon umfasst, insbesondere eine, die eine Aminosäuresequenz umfasst, die zu der in SEQ ID NO:3 angegebenen Aminosäuresequenz über deren Gesamtlänge zu mindestens 80% bevorzugt zu mindestens 90%, insbesondere zu 100% identisch ist und eine Aminosäuresubstitution an der Position 99 und eine Insertion von einer Aminosäure zwischen den Aminosäuren an Positionen 99 und 100 in der Zählung gemäß SEQ ID NO:3 aufweist, vorzugsweise ausgewählt aus S99A und/oder S99_G100lnsD; und/oder
(c) eine alkalische Protease aus *Bacillus lentus* DSM 5483 oder ein funktionales Fragment oder eine funktionale Variante davon umfasst, insbesondere eine, die eine Aminosäuresequenz aufweist, die zu der in SEQ ID NO:4 angegebenen Aminosäuresequenz über deren Gesamtlänge zu mindestens 80%, bevorzugt zu mindestens 90%, insbesondere zu 100% identisch ist und die optional mindestens eine Aminosäuresubstitution an einer, zwei, drei oder vier der folgenden Positionen 3, 4, 99 und 199 in der Zählung gemäß SEQ ID NO:4 aufweist, insbesondere die Aminosäuresubstitution R99E oder R99D, sowie optional zusätzlich mindestens eine oder zwei, vorzugsweise alle drei der Aminosäuresubstitutionen S3T, V4I und V199I; und/oder
(d) eine Aminosäuresequenz gemäß einer der SEQ ID NOs: 5-8 aufweist.

2. Reinigungsmittel nach Anspruch 1, **dadurch gekennzeichnet, dass** die erste Amylase eine Aminosäuresequenz umfasst, die zu der in SEQ ID NO:1 angegebenen Aminosäuresequenz über deren Gesamtlänge zu mindestens 80% identisch ist und optional mindestens eine Aminosäuresubstitution an einer der Positionen 172, 202, 208, 255 und 261 in der Zählung gemäß SEQ ID NO. 1 aufweist, insbesondere ausgewählt aus der Gruppe bestehend aus M202L, M202V, M202S, M202T, M202I, M202Q, M202W, S255N und R172Q.

3. Reinigungsmittel nach Anspruch 1 oder 2, **dadurch gekennzeichnet, dass** die zweite Amylase eine Aminosäuresequenz umfasst, die zu der in SEQ ID NO:2 angegebenen Aminosäuresequenz über deren Gesamtlänge zu mindestens 80% identisch ist und optional mindestens eine Aminosäuresubstitution an einer der Positionen 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 und 484 und/oder eine der Deletionen D183* and G184* in der Zählung gemäß SEQ ID NO:2 aufweist..

4. Reinigungsmittel nach Anspruch 3, **dadurch gekennzeichnet, dass** die zweite Amylase in der Zählung gemäß SEQ ID NO:2 Aminosäuresubstitutionen an drei oder mehr der Positionen 9, 26, 149, 182, 186, 202, 257, 295, 299, 323, 339 and 345 und optional eine oder mehrere, vorzugsweise alle, der Substitutionen und/oder Deletionen an den Positionen: 118, 183, 184, 195, 320 und 458, besonders bevorzugt R118K, D183*, G184*, N195F, R320K und/oder R458K, aufweist.

5. Reinigungsmittel nach Anspruch 3 oder 4, **dadurch gekennzeichnet, dass** die zweite Amylase in der Zählung gemäß SEQ ID NO:2 folgende Aminosäuresubstitutionen und/oder Deletionen aufweist:
(i) M9L + M323T;
(ii) M9L + M202L/T/V/I + M323T;
(iii) M9L + N195F + M202L/T/V/I + M323T;
(iv) M9L + R118K + D183* + G184* + R320K + M323T + R458K;
(v) M9L + R118K + D183* + G184* + M202L/T/V/I + R320K + M323T + R458K;
(vi) M9L + G149A + G182T + G186A + M202L + T257I + Y295F + N299Y + M323T + A339S + E345R;
(vii) M9L + G149A + G182T + G186A + M202I + T257I + Y295F + N299Y + M323T + A339S + E345R;
(viii) M9L + R118K + G149A + G182T + D183* + G184* + G186A + M202L + T257I + Y295F + N299Y + R320K + M323T + A339S + E345R + R458K;
(ix) M9L + R118K + G149A + G182T + D183* + G184* + G186A + N195F + M202L + T257I + Y295F + N299Y + R320K + M323T + A339S + E345R + R458K;
(x) M9L + R118K + G149A + G182T + D183* + G184* + G186A + M202I + T257I + Y295F + N299Y + R320K + M323T + A339S + E345R + R458K;
(xi) M9L + R118K + D183* + D184* + N195F + M202L + R320K + M323T + R458K;
(xii) M9L + R118K + D183* + D184* + N195F + M202T + R320K + M323T + R458K;
(xiii) M9L + R118K + D183* + D184* + N195F + M202I + R320K + M323T + R458K;
(xiv) M9L + R118K + D183* + D184* + N195F + M202V + R320K + M323T + R458K;
(xv) M9L + R118K + N150H + D183* + D184* + N195F + M202L + V214T + R320K + M323T + R458K; oder
(xvi) M9L + R118K + D183* + D184* + N195F + M202L + V214T + R320K + M323T + E345N + R458K.

6. Reinigungsmittel nach einem der Ansprüche 1 bis 5, **dadurch gekennzeichnet, dass** die mindestens eine Protease zwei oder mehr Proteasen gemäß Anspruch 1 umfasst, insbesondere eine Kombination der Protease gemäß Anspruch 1 (a) mit einer weiteren Protease gemäß Anspruch 1 (b) - (c).

7. Reinigungsmittel nach einem der Ansprüche 1 bis 6, **dadurch gekennzeichnet, dass** es eine erste Amylase und eine zweite Amylase in einem Massenverhältnis von 50:1 bis 1:50, vorzugsweise 30:1 bis 1:10, insbesondere von 20:1 bis 2:1, bevorzugt von 15:1 bis 3:1, besonders bevorzugt 12:1 bis 5:1, beispielsweise 10:1 (jeweils bezogen auf die Menge Aktivprotein Amylase 1 zu Amylase 2) enthält.

8. Reinigungsmittel nach einem der Ansprüche 1 bis 7, **dadurch gekennzeichnet, dass** der Gewichtsanteil jeder der Amylasen und optional jeder der Proteasen, bezogen auf das entsprechende aktive Protein, am Gesamtgewicht des Mittels jeweils von 1 x 10⁻⁸ bis 5 Gew.-%, bevorzugt 0,001 bis 2 Gew.-%, weiter bevorzugt von 0,005 bis 1,5 Gew.-%, noch weiter bevorzugt von 0,0075 bis 1 Gew.-%, beispielsweise von 0,01 bis 0,6 Gew.-%, beträgt.

9. Reinigungsmittel nach einem der Ansprüche 1 bis 8, **dadurch gekennzeichnet, dass** es mindestens einen weiteren Bestandteil, vorzugsweise mindestens zwei weitere Bestandteile, ausgewählt aus der Gruppe bestehend aus Gerüststoffen, Tensiden, Polymeren, Bleichmitteln, Bleichkatalysatoren, Bleichaktivatoren, Nicht-Protease- und Nicht-Amylase-Enzymen, Korrosionsinhibitoren, Glaskorrosionsinhibitoren, Desintegrationshilfsmitteln, Duftstoffen und Parfümträgern enthält.

10. Reinigungsmittel nach einem der Ansprüche 1 bis 9, **dadurch gekennzeichnet, dass** das Reinigungsmittel ein maschinelles Geschirrspülmittel ist und das
(1) maschinelle Geschirrspülmittel in fester Form vorliegt; und/oder
(2) maschinelle Geschirrspülmittel in vorportionierter Form vorliegt; und/oder
(3) maschinelle Geschirrspülmittel mehrere räumlich voneinander getrennte Zusammensetzungen aufweist.

11. Verwendung eines Reinigungsmittels nach einem der Ansprüche 1 bis 10 in einem maschinellen Geschirrspülverfahren.

12. Verfahren zur Reinigung von Geschirr in einer automatischen Geschirrspülmaschine, **dadurch gekennzeichnet, dass** ein Reinigungsmittel nach einem der Ansprüche 1 bis 10 während des Durchlaufens eines Geschirrspülprogramms vor Beginn des Hauptspülgangs oder im Verlaufe des Hauptspülgangs in den Innenraum einer Geschirrspülmaschine eindosiert wird.

13. Verwendung einer Kombination einer ersten und einer zweiten Amylase zur Verbesserung der Reinigungsleistung, insbesondere der Reinigungsleistung an enzymsensitiven Anschmutzungen, eines Reinigungsmittels, insbesondere eines Geschirrspülmittels, vorzugsweise eines maschinellen Geschirrspülmittels, **dadurch gekennzeichnet, dass**
a) die erste Amylase eine α-Amylase aus Bacillus sp. No. 707 oder ein funktionales Fragment oder eine funktionale Variante davon ist,
b) die zweite Amylase eine AA560 α-Amylase aus Bacillus sp. oder ein funktionales Fragment oder eine funktionale Variante davon ist, und
das Reinigungsmittel ferner mindestens eine Protease enthält, wobei die mindestens eine Protease
(a) ein Subtilisin 309 aus *Bacillus lentus* oder ein funktionales Fragment oder eine funktionale Variante davon umfasst, insbesondere eine, die eine Aminosäuresequenz umfasst, die zu der in SEQ ID NO:3 angegebenen Aminosäuresequenz über deren Gesamtlänge zu mindestens 80%, bevorzugt zu mindestens 90%, insbesondere zu 100% identisch ist und mindestens eine Aminosäuresubstitution an einer der Positionen 9, 15, 66, 212 und 239 in der Zählung gemäß SEQ ID NO:3 aufweist, vorzugsweise ausgewählt aus der Gruppe bestehend aus S9R, A15T, V66A, N212D und Q239R; und/oder
(b) ein Subtilisin 309 aus *Bacillus lentus* oder ein funktionales Fragment oder eine funktionale Variante davon umfasst, insbesondere eine, die eine Aminosäuresequenz umfasst, die zu der in SEQ ID NO:3 angegebenen Aminosäuresequenz über deren Gesamtlänge zu mindestens 80% bevorzugt zu mindestens 90%, insbesondere zu 100% identisch ist und eine Aminosäuresubstitution an der Position 99 und eine Insertion von einer Aminosäure zwischen den Aminosäuren an Positionen 99 und 100 in der Zählung gemäß SEQ ID NO:3 aufweist, vorzugsweise ausgewählt aus S99A und/oder S99_G100InsD; und/oder
(c) eine alkalische Protease aus *Bacillus lentus* DSM 5483 oder ein funktionales Fragment oder eine funktionale Variante davon umfasst, insbesondere eine, die eine Aminosäuresequenz aufweist, die zu der in SEQ ID NO:4 angegebenen Aminosäuresequenz über deren Gesamtlänge zu mindestens 80%, bevorzugt zu mindestens 90%, insbesondere zu 100% identisch ist und die optional mindestens eine Aminosäuresubstitution an einer, zwei, drei oder vier der folgenden Positionen 3, 4, 99 und 199 in der Zählung gemäß SEQ ID NO:4 aufweist, insbesondere die Aminosäuresubstitution R99E oder R99D, sowie optional zusätzlich mindestens eine oder zwei, vorzugsweise alle drei der Aminosäuresubstitutionen S3T, V4I und V199I; und/oder
(d) eine Aminosäuresequenz gemäß einer der SEQ ID NOs: 5-8 aufweist.

## Claims

1. A cleaning agent for hard surfaces, in particular a dishwashing detergent, preferably an automatic dishwashing detergent, **characterized in that**
- it comprises at least a first and a second amylase,
(a) the first amylase being an α-amylase from Bacillus sp. no. 707 or a functional fragment or a functional variant thereof,
(b) the second amylase being an AA560 α-amylase from Bacillus sp. or a functional fragment or a functional variant thereof, and
- it further contains at least one protease, the at least one protease
(a) comprising a subtilisin 309 from *Bacillus lentus* or a functional fragment or a functional variant thereof, in particular one which has an amino acid sequence which is at least 80%, preferably at least 90%, in particular 100%, identical to the amino acid sequence specified in SEQ ID NO:3 over the entire length thereof and has at least one amino acid substitution at one of the positions 9, 15, 66, 212 and 239 in the numbering according to SEQ ID NO:3, preferably selected from the group consisting of S9R, A15T, V66A, N212D and Q239R; and/or
(b) comprising a subtilisin 309 from *Bacillus lentus* or a functional fragment or a functional variant thereof, in particular one which comprises an amino acid sequence which is at least 80%, preferably at least 90%, in particular 100%, identical to the amino acid sequence specified in SEQ ID NO:3 over the entire length thereof and has an amino acid substitution at the position 99 and an insertion of an amino acid between the amino acids at positions 99 and 100 in the numbering according to SEQ ID NO:3, preferably selected from S99A and/or S99_G100InsD; and/or
(c) comprising an alkaline protease from *Bacillus lentus* DSM 5483 or a functional fragment or a functional variant thereof, in particular one which has an amino acid sequence which is at least 80%, preferably at least 90%, in particular 100%, identical to the amino acid sequence specified in SEQ ID NO:4 over the entire length thereof and optionally has at least one amino acid substitution at one, two, three or four of the following positions 3, 4, 99 and 199 in the numbering according to SEQ ID NO:4, in particular the amino acid substitution R99E or R99D, and optionally additionally at least one or two, preferably all three, of the amino acid substitutions S3T, V4I and V199I; and/or
(d) having an amino acid sequence according to one of SEQ ID NOs: 5-8.

2. The cleaning agent according to claim 1, **characterized in that** the first amylase comprises an amino acid sequence which is at least 80% identical to the amino acid sequence specified in SEQ ID NO:1 over the entire length thereof and optionally has at least one amino acid substitution at one of the positions 172, 202, 208, 255 and 261 in the numbering according to SEQ ID NO:1, in particular selected from the group consisting of M202L, M202V, M202S, M202T, M202I, M202Q, M202W, S255N and R172Q.

3. The cleaning agent according to claim 1 or 2, **characterized in that** the second amylase comprises an amino acid sequence which is at least 80% identical to the amino acid sequence specified in SEQ ID NO:2 over the entire length thereof and optionally has at least one amino acid substitution at one of the positions 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 and 484 and/or one of the deletions D183* and G184* in the numbering according to SEQ ID NO:2.

4. The cleaning agent according to claim 3, **characterized in that** the second amylase in the numbering according to SEQ ID NO:2 has an amino acid sequence at three or more of the positions 9, 26, 149, 182, 186, 202, 257, 295, 299, 323, 339 and 345 and optionally one or more, preferably all, of the substitutions and/or deletions at the positions: 118, 183, 184, 195, 320 and 458, particularly preferably R118K, D183*, G184*, N195F, R320K and/or R458K.

5. The cleaning agent according to claim 3 or 4, **characterized in that** the second amylase in the numbering according to SEQ ID NO:2 has the following amino acid substitutions and/or deletions:
(i) M9L + M323T;
(ii) M9L + M202L/T/V/I + M323T;
(iii) M9L + N195F + M202L/T/V/I + M323T;
(iv) M9L + R118K + D183* + G184* + R320K + M323T + R458K;
(v) M9L + R118K + D183* + G184* + M202L/T/V/I + R320K + M323T + R458K;
(vi) M9L + G149A + G182T + G186A + M202L + T257I + Y295F + N299Y + M323T + A339S + E345R;
(vii) M9L + G149A + G182T + G186A + M202I + T257I + Y295F + N299Y + M323T + A339S + E345R;
(viii) M9L + R118K + G149A + G182T + D183* + G184* + G186A + M202L + T257I + Y295F + N299Y + R320K + M323T + A339S + E345R + R458K;
(ix) M9L + R118K + G149A + G182T + D183* + G184* + G186A + N195F + M202L + T257I + Y295F + N299Y + R320K + M323T + A339S + E345R + R458K;
(x) M9L + R118K + G149A + G182T + D183* + G184* + G186A + M202I + T257I + Y295F + N299Y + R320K + M323T + A339S + E345R + R458K;
(xi) M9L + R118K + D183* + D184* + N195F + M202L + R320K + M323T + R458K;
(xii) M9L + R118K + D183* + D184* + N195F + M202T + R320K + M323T + R458K;
(xiii) M9L + R118K + D183* + D184* + N195F + M202I + R320K + M323T + R458K;
(xiv) M9L + R118K + D183* + D184* + N195F + M202V + R320K + M323T + R458K;
(xv) M9L + R118K + N150H + D183* + D184* + N195F + M202L + V214T + R320K + M323T + R458K; or
(xvi) M9L + R118K + D183* + D184* + N195F + M202L +V214T + R320K + M323T + E345N + R458K.

6. The cleaning agent according to one of claims 1 to 5, **characterized in that** the at least one protease comprises two or more proteases according to claim 1, in particular a combination of the protease according to claim 1 (a) with another protease according to claim 1 (b) - (c).

7. The cleaning agent according to one of claims 1 to 6, **characterized in that** it contains a first amylase and a second amylase in a mass ratio of from 50:1 to 1:50, preferably 30:1 to 1:10, in particular 20:1 to 2:1, preferably 15:1 to 3:1, particularly preferably 12:1 to 5:1, for example 10:1 (based on the amount of active protein amylase 1 to amylase 2 in each case).

8. The cleaning agent according to one of claims 1 to 7, **characterized in that** the weight proportion of each of the amylases and optionally of each of the proteases, based on the corresponding active protein, with respect to the total weight of the agent is of from 1 x 10⁻⁸ to 5 wt. %, preferably 0.001 to 2 wt.%, more preferably of from 0.005 to 1.5 wt.%, even more preferably of from 0.0075 to 1 wt.%, for example of from 0.01 to 0.6 wt.%.

9. The cleaning agent according to one of claims 1 to 8, **characterized in that** it contains at least one further component, preferably at least two further components, selected from the group consisting of builders, surfactants, polymers, bleaching agents, bleach catalysts, bleach activators, non-protease and non-amylase enzymes, corrosion inhibitors, glass corrosion inhibitors, disintegration auxiliaries, fragrances and perfume carriers.

10. The cleaning agent according to one of claims 1 to 9, **characterized in that** the cleaning agent is an automatic dishwashing detergent and
(1) the automatic dishwashing detergent is in solid form; and/or
(2) the automatic dishwashing detergent is in a pre-portioned form; and/or
(3) the automatic dishwashing detergent has a plurality of compositions that are spatially separated from one another.

11. The use of a cleaning agent according to one of claims 1 to 10 in an automatic dishwashing method.

12. A method for cleaning dishes in an automatic dishwasher, **characterized in that** a cleaning agent according to one of claims 1 to 10 is dispensed into the interior of a dishwasher while a dishwashing program is running, before the main washing cycle begins or during the main washing cycle.

13. The use of a combination of a first and a second amylase for improving cleaning performance, in particular the cleaning performance with respect to enzyme-sensitive stains, of a cleaning agent, in particular of a dishwashing detergent, preferably an automatic dishwashing detergent, **characterized in that**
(a) the first amylase is an α-amylase from Bacillus sp. no. 707 or a functional fragment or a functional variant thereof,
(b) the second amylase is an AA560 α-amylase from Bacillus sp. or a functional fragment or a functional variant thereof, and
the cleaning agent further contains at least one protease, the at least one protease
(a) comprising a subtilisin 309 from *Bacillus lentus* or a functional fragment or a functional variant thereof, in particular one which has an amino acid sequence which is at least 80%, preferably at least 90%, in particular 100%, identical to the amino acid sequence specified in SEQ ID NO:3 over the entire length thereof and has at least one amino acid substitution at one of the positions 9, 15, 66, 212 and 239 in the numbering according to SEQ ID NO:3, preferably selected from the group consisting of S9R, A15T, V66A, N212D and Q239R; and/or
(b) comprising a subtilisin 309 from *Bacillus lentus* or a functional fragment or a functional variant thereof, in particular one which comprises an amino acid sequence which is at least 80%, preferably at least 90%, in particular 100%, identical to the amino acid sequence specified in SEQ ID NO:3 over the entire length thereof and has an amino acid substitution at the position 99 and an insertion of an amino acid between the amino acids at positions 99 and 100 in the numbering according to SEQ ID NO:3, preferably selected from S99A and/or S99_G100InsD; and/or
(c) comprising an alkaline protease from *Bacillus lentus* DSM 5483 or a functional fragment or a functional variant thereof, in particular one which has an amino acid sequence which is at least 80%, preferably at least 90%, in particular 100%, identical to the amino acid sequence specified in SEQ ID NO:4 over the entire length thereof and optionally has at least one amino acid substitution at one, two, three or four of the following positions 3, 4, 99 and 199 in the numbering according to SEQ ID NO:4, in particular the amino acid substitution R99E or R99D, and optionally additionally at least one or two, preferably all three, of the amino acid substitutions S3T, V4I and V199I; and/or
(d) having an amino acid sequence according to one of SEQ ID NOs: 5-8.

## Revendications

1. Produit de nettoyage pour surfaces dures, en particulier un produit vaisselle, de préférence un produit pour lave-vaisselle, **caractérisé en ce**
- **qu'**il comprend au moins une première et une seconde amylase,
(a) la première amylase étant une α-amylase de Bacillus sp. n° 707, un fragment fonctionnel ou une variante de celle-ci,
(b) la seconde amylase étant une α-amylase AA560 de Bacillus sp., un fragment fonctionnel ou une variante fonctionnelle de celle-ci, et
- **qu'**elle contient en outre au moins une protéase, ladite au moins une protéase
(a) comprenant une subtilisine 309 de *Bacillus lentus*, un fragment fonctionnel ou une variante fonctionnelle de celle-ci, en particulier une variante présentant une séquence d'acides aminés identique sur toute sa longueur à raison d'au moins 80 %, de préférence d'au moins 90 %, en particulier d'au moins 100 %, à la séquence d'acides aminés indiquée dans SEQ ID NO:3, et présentant au moins une substitution d'acide aminé à l'une des positions 9, 15, 66, 212 et 239 dans le comptage conforme à la SEQ ID NO:3, de préférence choisie dans le groupe constitué par S9R, A15T, V66A, N212D et Q239R ; et/ou
(b) comprenant une subtilisine 309 de *Bacillus lentus*, un fragment fonctionnel ou une variante fonctionnelle de celle-ci, en particulier une variante présentant une séquence d'acides aminés identique sur toute sa longueur à raison d'au moins 80 %, de préférence d'au moins 90 %, en particulier d'au moins 100 %, à la séquence d'acides aminés indiquée dans SEQ ID NO:3, et présentant au moins une substitution d'acide aminé en position 99 et une insertion d'un acide aminé entre les acides aminés aux positions 99 et 100 dans le comptage conforme à la SEQ ID NO:3, de préférence choisie parmi S99A et/ou S99_G100InsD ; et/ou
(c) comprenant une protéase alcaline de *Bacillus lentus* DSM 5483, un fragment fonctionnel ou une variante fonctionnelle de celle-ci, en particulier une variante présentant une séquence d'acides aminés identique sur toute sa longueur à raison d'au moins 80 %, de préférence d'au moins 90 %, en particulier d'au moins 100 % à la séquence d'acides aminés indiquée dans SEQ ID NO:4, et présentant éventuellement au moins une substitution d'acide aminé à une, deux, trois ou quatre des positions 3, 4, 99 et 199 dans le comptage conforme à la SEQ ID NO:4, en particulier la substitution d'acides aminés R99E ou R99D, et éventuellement en outre au moins une ou deux, de préférence les trois substitutions d'acides aminés S3T, V4I et V199I ; et/ou
(d) présentant une séquence d'acides aminés conforme à l'une des SEQ ID NO: 5 à 8.

2. Produit de nettoyage selon la revendication 1, **caractérisé en ce que** la première amylase comprend une séquence d'acides aminés identique sur toute sa longueur à raison d'au moins 80 % à la séquence d'acides aminés indiquée dans SEQ ID NO:1, et présentant éventuellement au moins une substitution d'acides aminés à l'une des positions 172, 202, 208, 255 et 261 dans le comptage conforme à la SEQ ID NO: 1, en particulier choisie dans le groupe constitué par M202L, M202V, M202S, M202T, M202I, M202Q, M202W, S255N et R172Q.

3. Produit de nettoyage selon la revendication 1 ou 2, **caractérisé en ce que** la seconde amylase comprend une séquence d'acides aminés identique sur toute sa longueur à raison d'au moins 80 % à la séquence d'acides aminés indiquée dans SEQ ID NO:2, et éventuellement au moins une substitution d'acide aminé à l'une des positions 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 et 484 et/ou l'une des délétions D183* et G184* dans le comptage conforme à la SEQ ID NO:2.

4. Produit de nettoyage selon la revendication 3, **caractérisé en ce que** la seconde amylase présente, dans le comptage conforme à la SEQ ID NO:2, des substitutions d'acides aminés à au moins trois positions parmi les positions 9, 26, 149, 182, 186, 202, 257, 295, 299, 323, 339 et 345, et éventuellement une ou plusieurs des substitutions et/ou des délétions, de préférence toutes, aux positions : 118, 183, 184, 195, 320 et 458, particulièrement de préférence R118K, D183*, G184*, N195F, R320K et/ou R458K.

5. Produit de nettoyage selon la revendication 3 ou 4, **caractérisé en ce que** la seconde amylase présente, dans le comptage conforme à la SEQ ID NO:2, les substitutions et/ou délétions d'acides aminés suivantes :
(i) M9L + M323T ;
(ii) M9L + M202L/T/V/I + M323T ;
(iii) M9L + N195F + M202L/T/V/I + M323T ;
(iv) M9L + R118K + D183* + G184* + R320K + M323T + R458K ;
(v) M9L + R118K + D183* + G184* + M202L/T/V/I + R320K + M323T + R458K ;
(vi) M9L + G149A + G182T + G186A + M202L + T257I + Y295F + N299Y + M323T + A339S + E345R ;
(vii) M9L + G149A + G182T + G186A + M202I + T257I + Y295F + N299Y + M323T + A339S + E345R ;
(viii) M9L + R118K + G149A + G182T + D183* + G184* + G186A + M202L + T257I + Y295F + N299Y + R320K + M323T + A339S + E345R + R458K ;
(ix) M9L + R118K + G149A + G182T + D183* + G184* + G186A + N195F + M202L + T257I + Y295F + N299Y + R320K + M323T + A339S + E345R + R458K ;
(x) M9L + R118K + G149A + G182T + D183* + G184* + G186A + M202I + T257I + Y295F + N299Y + R320K + M323T + A339S + E345R + R458K ;
(xi) M9L + R118K + D183* + D184* + N195F + M202L + R320K + M323T + R458K ;
(xii) M9L + R118K + D183* + D184* + N195F + M202T + R320K + M323T + R458K ;
(xiii) M9L + R118K + D183* + D184* + N195F + M202I + R320K + M323T + R458K ;
(xiv) M9L + R118K + D183* + D184* + N195F + M202V + R320K + M323T + R458K ;
(xv) M9L + R118K + N150H + D183* + D184* + N195F + M202L + V214T + R320K + M323T + R458K ; ou
(xvi) M9L + R118K + D183* + D184* + N195F + M202L + V214T + R320K + M323T + E345N + R458K.

6. Produit de nettoyage selon les revendications 1 à 5, **caractérisé en ce que** l'au moins une protéase comprend au moins deux protéases selon la revendication 1, en particulier une combinaison de la protéase selon la revendication 1 (a) avec une autre protéase selon la revendication 1 (b) à (c).

7. Produit de nettoyage selon l'une des revendications 1 à 6, **caractérisé en ce qu'**il contient une première amylase et une seconde amylase dans un rapport massique de 50:1 à 1:50, de préférence de 30:1 à 1:10, en particulier de 20:1 à 2:1, préférablement de 15:1 à 3:1, de manière particulièrement préférée de 12:1 à 5:1, par exemple de 10:1 (dans chaque cas par rapport à la quantité de protéine active d'amylase 1 à amylase 2).

8. Produit de nettoyage selon l'une des revendications 1 à 7, **caractérisé en ce que** la proportion en poids de chacune des amylases et éventuellement de chacune des protéases, par rapport à la protéine active correspondante du poids total de l'agent, représente respectivement 1 x 10⁻⁸ à 5 % en poids, de préférence 0,001 à 2 % en poids, plus préférablement 0,005 à 1,5 % en poids, plus préférablement encore 0,0075 à 1 % en poids, par exemple 0,01 à 0,6 % en poids.

9. Produit de nettoyage selon l'une des revendications 1 à 8, **caractérisé en ce qu'**il contient au moins un autre constituant, de préférence au moins deux autres constituants choisis dans le groupe constitué par les adjuvants, les tensioactifs, les polymères, les agents de blanchiment, les catalyseurs de blanchiment, les activateurs de blanchiment, les enzymes non-protéases et non-amylases, les inhibiteurs de corrosion, les inhibiteurs de corrosion du verre, les adjuvants de désintégration, les parfums et les supports de parfum.

10. Produit de nettoyage selon l'une des revendications 1 à 9, **caractérisé en ce qu'**il est un produit pour lave-vaisselle, et **en ce que** le
(1) produit pour lave-vaisselle se présente sous forme solide ; et/ou
(2) le produit pour lave-vaisselle se présente sous forme pré-portionnée ; et/ou
(3) le produit pour lave-vaisselle présente plusieurs compositions séparées physiquement les unes des autres.

11. Utilisation d'un produit de nettoyage selon l'une des revendications 1 à 10 dans un procédé de lavage de la vaisselle en machine.

12. Procédé de lavage de la vaisselle dans un lave-vaisselle automatique, **caractérisé en ce qu'**un produit de nettoyage selon l'une des revendications 1 à 10 est dosé à l'intérieur du lave-vaisselle pendant son fonctionnement avant le début du cycle de lavage principal ou au cours de celui-ci.

13. Utilisation d'une combinaison d'une première et d'une seconde amylase pour améliorer le pouvoir nettoyant, en particulier le pouvoir nettoyant sur les salissures sensibles aux enzymes ; utilisation d'un produit de nettoyage, en particulier d'un produit vaisselle, de préférence un produit pour lave-vaisselle, **caractérisée en ce que**
(a) la première amylase est une α-amylase de Bacillus sp. n° 707, un fragment fonctionnel ou une variante de celle-ci,
(b) la seconde amylase est une α-amylase AA560 de Bacillus sp., un fragment fonctionnel ou une variante fonctionnelle de celle-ci, et
le produit détergent contient en outre au moins une protéase, ladite au moins une protéase
(a) comprenant une subtilisine 309 de *Bacillus lentus*, un fragment fonctionnel ou une variante fonctionnelle de celle-ci, en particulier une variante présentant une séquence d'acides aminés identique sur toute sa longueur à raison d'au moins 80 %, de préférence d'au moins 90 %, en particulier d'au moins 100 %, à la séquence d'acides aminés indiquée dans SEQ ID NO:3, et présentant au moins une substitution d'acide aminé à l'une des positions 9, 15, 66, 212 et 239 dans le comptage conforme à la SEQ ID NO:3, de préférence choisie dans le groupe constitué par S9R, A15T, V66A, N212D et Q239R ; et/ou
(b) comprenant une subtilisine 309 de *Bacillus lentus*, un fragment fonctionnel ou une variante fonctionnelle de celle-ci, en particulier une variante présentant une séquence d'acides aminés identique sur toute sa longueur à raison d'au moins 80 %, de préférence d'au moins 90 %, en particulier d'au moins 100 %, à la séquence d'acides aminés indiquée dans SEQ ID NO:3, et présentant au moins une substitution d'acide aminé en position 99 et une insertion d'un acide aminé entre les acides aminés aux positions 99 et 100 dans le comptage conforme à la SEQ ID NO:3, de préférence choisie parmi S99A et/ou S99_G100lnsD ; et/ou
(c) comprenant une protéase alcaline de *Bacillus lentus* DSM 5483, un fragment fonctionnel ou une variante fonctionnelle de celle-ci, en particulier une variante présentant une séquence d'acides aminés identique sur toute sa longueur à raison d'au moins 80 %, de préférence d'au moins 90 %, en particulier d'au moins 100 % à la séquence d'acides aminés indiquée dans SEQ ID NO:4, et présentant éventuellement au moins une substitution d'acide aminé à une, deux, trois ou quatre des positions 3, 4, 99 et 199 dans le comptage conforme à la SEQ ID NO:4, en particulier la substitution d'acides aminés R99E ou R99D, et éventuellement en outre au moins une ou deux, de préférence les trois substitutions d'acides aminés S3T, V4I et V199I ; et/ou
(d) présentant une séquence d'acides aminés conforme à l'une des SEQ ID NO: 5 à 8.
